# EUROPEAN PATENT SPECIFICATION

(11) **EP 2 265 632 B1**
(45) Date of publication and mention of the grant of the patent: **07.01.2015**
(21) Application number: 09726122.6
(22) Date of filing: 27.03.2009
(51) Int. Cl.: C07K 14/16

(54) **CD4-RELATED POLYPEPTIDES AND METHODS OF USE**
MIT CD4 VERWANDTE POLYPEPTIDE UND ANWENDUNGSVERFAHREN
POLYPEPTIDES APPARENTÉS À CD4 ET PROCÉDÉS D UTILISATION

(30) Priority: 27.03.2008 US 40128 P
(43) Date of publication of application: 29.12.2010
(73) Proprietor: Elevation Biotech (Pty) Ltd., 2193 Johannesburg (ZA)
(72) Inventor: NAPIER, Grant, Bredell, 2196 Johannesburg (ZA); CAPOVILLA, Alexio, Dr., 2192 Johannesburg (ZA); MENDELOW, Barry, 7357 Western Cape (ZA)
(74) Representative: Kremer, Simon Mark
(86) International application number: PCT/IB2009/051287
(87) International publication number: WO 2009/118710

(56) References cited:
- WO-A2-2004/048414
- ZAGURY J F ET AL: "Identification of CD4 and major histocompatibility complex functional peptide sites and their homology with oligopeptides from human immunodeficiency virus type 1 glycoprotein gp120: role in AIDS pathogenesis." PROCEEDINGS OF THE NATIONAL ACADEMY OF SCIENCES OF THE UNITED STATES OF AMERICA 15 AUG 1993, vol. 90, no. 16, 15 August 1993 (1993-08-15), pages 7573-7577, XP002539047 ISSN: 0027-8424
- DATABASE MEDLINE [Online] US NATIONAL LIBRARY OF MEDICINE (NLM), BETHESDA, MD, US 13 August 2010 CERUTTI NICHOLE ET AL: 'Stabilization of HIV-1 gp120-CD4 receptor complex through targeted interchain disulfide exchange.' Database accession no. NLM20538591 & THE JOURNAL OF BIOLOGICAL CHEMISTRY 13 AUG 2010 LNKD- PUBMED:20538591, vol. 285, no. 33, 13 August 2010 (2010-08-13), pages 25743-25752, ISSN: 1083-351X

### SEQUENCE LISTING

<110> Elevation Biotech Pty Ltd.
   National Health Laboratory Service
   University of Witwatersrand
<120> CD4 POLYPEPTIDES AND METHODS OF USE
<130> 23795-0003WO1
<150> US 61/040,128
   <151> 2008-03-27
<160> 33
<170> FastSEQ for Windows Version 4.0
<210> 1
   <211> 7
   <212> PRT
   <213> Artificial Sequence
<220>
<223> Synthetically generated peptide
<220>
   <221> VARIANT
   <222> 1, 3
   <223> Xaa = any amino acid
<220>
   <221> VARIANT
   <222> 5
   <223> Xaa = Trp, Phe, or Tyr
<400> 1
   Xaa Arg Xaa Leu Xaa Asp Gln
   1   5
<210> 2
   <211> 7
   <212> PRT
   <213> Artificial Sequence
<220>
   <223> Synthetically generated peptide
<400> 2
   Arg Arg Cys Leu Trp Asp Gln
   1   5
<210> 3
   <211> 7
   <212> PRT
   <213> Artificial Sequence
<220>
   <223> Synthetically generated peptide
<220>
   <221> VARIANT
   <222> 3
   <223> Xaa = selenocysteine
<400> 3
   Arg Arg Xaa Leu Trp Asp Gln
   1   5
<210> 4
   <211> 7
   <212> PRT
   <213> Artificial Sequence
<220>
   <223> Synthetically generated peptide
<400> 4
   Cys Arg Ser Leu Trp Asp Gln
   1   5
<210> 5
   <211> 7
   <212> PRT
   <213> Artificial Sequence
<220>
   <223> Synthetically generated peptide
<220>
   <221> VARIANT
   <222> 1
   <223> Xaa = selenocysteine
<400> 5
   Xaa Arg Ser Leu Trp Asp Gln
   1 5
<210> 6
   <211> 7
   <212> PRT
   <213> Artificial Sequence
<220>
   <223> Synthetically generated peptide
<400> 6
   Cys Arg Cys Leu Trp Asp Gln
   1 5
<210> 7
   <211> 7
   <212> PRT
   <213> Artificial Sequence
<220>
   <223> Synthetically generated peptide
<220>
   <221> VARIANT
   <222> 1, 3
   <223> Xaa = selenocysteine
<400> 7
   Xaa Arg Xaa Leu Trp Asp Gln
   1 5
<210> 8
   <211> 7
   <212> PRT
   <213> Artificial Sequence
<220>
   <223> Synthetically generated peptide
<220>
   <221> VARIANT
   <222> 3
   <223> Xaa = selenocysteine
<400> 8
   Cys Arg Xaa Leu Trp Asp Gln
   1 5
<212> PRT
   <213> Artificial Sequence
<220>
   <223> Synthetically generated peptide
<220>
   <221> VARIANT
   <222> 1
   <223> Xaa = selenocysteine
<400> 9
<210> 10
   <211> 7
   <212> PRT
   <213> Artificial Sequence
<220>
   <223> Synthetically generated peptide
<400> 10
   Arg Arg Ser Leu Trp Asp Gln
   1 5
<210> 11
   <211> 5
   <212> PRT
   <213> Homo sapiens
<400> 11
   Arg Arg Cys Leu Trp
   1 5
<210> 12
   <211> 5
   <212> PRT
   <213> Artificial Sequence
<220>
   <223> Synthetically generated peptide
<220>
   <221> VARIANT
   <222> 1, 3
   <223> Xaa = any amino acid
<220>
   <221> VARIANT
   <222> 5
   <223> Xaa = Trp, Phe, or Tyr
<400> 12
   Xaa Arg Xaa Leu Xaa
   1 5
<210> 13
   <211> 57
   <212> PRT
   <213> Homo sapiens
<400> 13
   Lys Lys Val Val Leu Gly Lys Lys Gly Asp Thr Val Glu Leu Thr Cys
   1 5 10 15
   Thr Ala Ser Gln Lys Lys Ser Ile Gln Phe His Trp Lys Asn Ser Asn
   20 25 30
   Gln Ile Lys Ile Leu Gly Asn Gln Gly Ser Phe Leu Thr Lys Gly Pro
   35 40 45
   Ser Lys Leu Asn Asp Arg Ala Asp Ser
   50 55
<210> 14
   <211> 82
   <212> PRT
   <213> Homo sapiens
<400> 14
   Met Asn Arg Gly Val Pro Phe Arg His Leu Leu Leu Val Leu Gln Leu
   1 5 10 15
   Ala Leu Leu Pro Ala Ala Thr Gln Gly Lys Lys Val Val Leu Gly Lys
   20 25 30
   Lys Gly Asp Thr Val Glu Leu Thr Cys Thr Ala Ser Gln Lys Lys Ser
   35 40 45
   Ile Gln Phe His Trp Lys Asn Ser Asn Gln Ile Lys Ile Leu Gly Asn
   50 55 60
   Gln Gly Ser Phe Leu Thr Lys Gly Pro Ser Lys Leu Asn Asp Arg Ala
   65 70 75 80
   Asp Ser
<210> 15
   <211> 8
   <212> PRT
   <213> Artificial Sequence
<220>
   <223> Synthetically generated peptide
<220>
   <221> VARIANT
   <222> 4
   <223> Xaa = Cysteine or selenocysteine
<400> 15
   Ser Arg Arg Xaa Leu Trp Asp Gln
   1 5
<210> 16
   <211> 9
   <212> PRT
   <213> Artificial Sequence
<220>
   <223> Synthetically generated peptide
<220>
   <221> VARIANT
   <222> 5
   <223> Xaa = Cysteine or selenocysteine
<400> 16
<210> 17
   <211> 10
   <212> PRT
   <213> Artificial Sequence
<220>
   <223> Synthetically generated peptide
<220>
   <221> VARIANT
   <222> 6
   <223> Xaa = Cysteine or selenocysteine
<400> 17
   Ala Asp Ser Arg Arg Xaa Leu Trp Asp Gln
   1 5 10
<210> 18
   <211> 369
   <212> PRT
   <213> Homo sapiens
<400> 18
   Gly Asn Phe Pro Leu Ile Ile Lys Asn Leu Lys Ile Glu Asp Ser Asp
   1 5 10 15
   Thr Tyr Ile Cys Glu Val Glu Asp Gln Lys Glu Glu Val Gln Leu Leu
   20 25 30
   Val Phe Gly Leu Thr Ala Asn Ser Asp Thr His Leu Leu Gln Gly Gln
   35 40 45
   Ser Leu Thr Leu Thr Leu Glu Ser Pro Pro Gly Ser Ser Pro Ser Val
   50 55 60
   Gln Cys Arg Ser Pro Arg Gly Lys Asn Ile Gln Gly Gly Lys Thr Leu
   65 70 75 80
   Ser Val Ser Gln Leu Glu Leu Gln Asp Ser Gly Thr Trp Thr Cys Thr
   85 90 95
   Val Leu Gln Asn Gln Lys Lys Val Glu Phe Lys Ile Asp Ile Val Val
   100 105 110
   Leu Ala Phe Gln Lys Ala Ser Ser Ile Val Tyr Lys Lys Glu Gly Glu
   115 120 125
   Gln Val Glu Phe Ser Phe Pro Leu Ala Phe Thr Val Glu Lys Leu Thr
   130 135 140
   Gly Ser Gly Glu Leu Trp Trp Gln Ala Glu Arg Ala Ser Ser Ser Lys
   145 150 155 160
   Ser Trp Ile Thr Phe Asp Leu Lys Asn Lys Glu Val Ser Val Lys Arg
   165 170 175
   Val Thr Gln Asp Pro Lys Leu Gln Met Gly Lys Lys Leu Pro Leu His
   180 185 190
   Leu Thr Leu Pro Gln Ala Leu Pro Gln Tyr Ala Gly Ser Gly Asn Leu
   195 200 205
   Thr Leu Ala Leu Glu Ala Lys Thr Gly Lys Leu His Gln Glu Val Asn
   210 215 220
   Leu Val Val Met Arg Ala Thr Gln Leu Gln Lys Asn Leu Thr Cys Glu
   225 230 235 240
   Val Trp Gly Pro Thr Ser Pro Lys Leu Met Leu Ser Leu Lys Leu Glu
   245 250 255
   Asn Lys Glu Ala Lys Val Ser Lys Arg Glu Lys Ala Val Trp Val Leu
   260 265 270
   Asn Pro Glu Ala Gly Met Trp Gln Cys Leu Leu Ser Asp Ser Gly Gln
   275 280 285
   Val Leu Leu Glu Ser Asn Ile Lys Val Leu Pro Thr Trp Ser Thr Pro
   290 295 300
   Val Gln Pro Met Ala Leu Ile Val Leu Gly Gly Val Ala Gly Leu Leu
   305 310 315 320
   Leu Phe Ile Gly Leu Gly Ile Phe Phe Cys Val Arg Cys Arg His Arg
   325 330 335
   Arg Arg Gln Ala Glu Arg Met Ser Gln Ile Lys Arg Leu Leu Ser Glu
   340 345 350
   Lys Lys Thr Cys Gln Cys Pro His Arg Phe Gln Lys Thr Cys Ser Pro
   355 360 365
   Ile
<210> 19
   <211> 8
   <212> PRT
   <213> Artificial Sequence
<220>
   <223> Synthetically generated peptide
<220>
   <221> VARIANT
   <222> 3
   <223> Xaa = Cysteine or selenocysteine
<400> 19
   Arg Arg Xaa Leu Trp Asp Gln Gly
   1 5
<210> 20
   <211> 9
   <212> PRT
   <213> Artificial Sequence
<220>
   <223> Synthetically generated peptide
<220>
   <221> VARIANT
   <222> 3
   <223> Xaa = Cysteine or selenocysteine
<400> 20
   Arg Arg Xaa Leu Trp Asp Gln Gly Asn
   1 5
<210> 21
   <211> 10
   <212> PRT
   <213> Artificial Sequence
<220>
   <223> Synthetically generated peptide
<220>
   <221> VARIANT
   <222> 3
   <223> Xaa = Cysteine or selenocysteine
<400> 21
   Arg Arg Xaa Leu Trp Asp Gln Gly Asn Phe
   1 5 10
<210> 22
   <211> 458
   <212> PRT
   <213> Homo sapiens
<400> 22
   Met Asn Arg Gly Val Pro Phe Arg His Leu Leu Leu Val Leu Gln Leu
   1 5 10 15
   Ala Leu Leu Pro Ala Ala Thr Gln Gly Lys Lys Val Val Leu Gly Lys
   20 25 30
   Lys Gly Asp Thr Val Glu Leu Thr Cys Thr Ala Ser Gln Lys Lys Ser
   35 40 45
   Ile Gln Phe His Trp Lys Asn Ser Asn Gln Ile Lys Ile Leu Gly Asn
   50 55 60
   Gln Gly Ser Phe Leu Thr Lys Gly Pro Ser Lys Leu Asn Asp Arg Ala
   65 70 75 80
   Asp Ser Arg Arg Ser Leu Trp Asp Gln Gly Asn Phe Pro Leu Ile Ile
   85 90 95
   Lys Asn Leu Lys Ile Glu Asp Ser Asp Thr Tyr Ile Cys Glu Val Glu
   100 105 110
   Asp Gln Lys Glu Glu Val Gln Leu Leu Val Phe Gly Leu Thr Ala Asn
   115 120 125
   Ser Asp Thr His Leu Leu Gln Gly Gln Ser Leu Thr Leu Thr Leu Glu
   130 135 140
   Ser Pro Pro Gly Ser Ser Pro Ser Val Gln Cys Arg Ser Pro Arg Gly
   145 150 155 160
   Lys Asn Ile Gln Gly Gly Lys Thr Leu Ser Val Ser Gln Leu Glu Leu
   165 170 175
   Gln Asp Ser Gly Thr Trp Thr Cys Thr Val Leu Gln Asn Gln Lys Lys
   180 185 190
   Val Glu Phe Lys Ile Asp Ile Val Val Leu Ala Phe Gln Lys Ala Ser
   195 200 205
   Ser Ile Val Tyr Lys Lys Glu Gly Glu Gln Val Glu Phe Ser Phe Pro
   210 215 220
   Leu Ala Phe Thr Val Glu Lys Leu Thr Gly Ser Gly Glu Leu Trp Trp
   225 230 235 240
   Gln Ala Glu Arg Ala Ser Ser Ser Lys Ser Trp Ile Thr Phe Asp Leu
   245 250 255
   Lys Asn Lys Glu Val Ser Val Lys Arg Val Thr Gln Asp Pro Lys Leu
   260 265 270
   Gln Met Gly Lys Lys Leu Pro Leu His Leu Thr Leu Pro Gln Ala Leu
   275 280 285
   Pro Gln Tyr Ala Gly Ser Gly Asn Leu Thr Leu Ala Leu Glu Ala Lys
   290 295 300
   Thr Gly Lys Leu His Gln Glu Val Asn Leu Val Val Met Arg Ala Thr
   305 310 315 320
   Gln Leu Gln Lys Asn Leu Thr Cys Glu Val Trp Gly Pro Thr Ser Pro
   325 330 335
   Lys Leu Met Leu Ser Leu Lys Leu Glu Asn Lys Glu Ala Lys Val Ser
   340 345 350
   Lys Arg Glu Lys Ala Val Trp Val Leu Asn Pro Glu Ala Gly Met Trp
   355 360 365
   Gln Cys Leu Leu Ser Asp Ser Gly Gln Val Leu Leu Glu Ser Asn Ile
   370 375 380
   Lys Val Leu Pro Thr Trp Ser Thr Pro Val Gln Pro Met Ala Leu Ile
   385 390 395 400
   Val Leu Gly Gly Val Ala Gly Leu Leu Leu Phe Ile Gly Leu Gly Ile
   405 410 415
   Phe Phe Cys Val Arg Cys Arg His Arg Arg Arg Gln Ala Glu Arg Met
   420 425 430
   Ser Gln Ile Lys Arg Leu Leu Ser Glu Lys Lys Thr Cys Gln Cys Pro
   435 440 445
   His Arg Phe Gln Lys Thr Cys Ser Pro Ile
   450 455
<210> 23
   <211> 458
   <212> PRT
   <213> Homo sapiens
<220>
   <221> MUTANT
<222> 85
   <223> Serine to Cysteine substitution
<400> 23
   Met Asn Arg Gly Val Pro Phe Arg His Leu Leu Leu Val Leu Gln Leu
   1 5 10 15
   Ala Leu Leu Pro Ala Ala Thr Gln Gly Lys Lys Val Val Leu Gly Lys
   20 25 30
   Lys Gly Asp Thr Val Glu Leu Thr Cys Thr Ala Ser Gln Lys Lys Ser
   35 40 45
   Ile Gln Phe His Trp Lys Asn Ser Asn Gln Ile Lys Ile Leu Gly Asn
   50 55 60
   Gln Gly Ser Phe Leu Thr Lys Gly Pro Ser Lys Leu Asn Asp Arg Ala
   65 70 75 80
   Asp Ser Arg Arg Cys Leu Trp Asp Gln Gly Asn Phe Pro Leu Ile Ile
   85 90 95
   Lys Asn Leu Lys Ile Glu Asp Ser Asp Thr Tyr Ile Cys Glu Val Glu
   100 105 110
   Asp Gln Lys Glu Glu Val Gln Leu Leu Val Phe Gly Leu Thr Ala Asn
   115 120 125
   Ser Asp Thr His Leu Leu Gln Gly Gln Ser Leu Thr Leu Thr Leu Glu
   130 135 140
   Ser Pro Pro Gly Ser Ser Pro Ser Val Gln Cys Arg Ser Pro Arg Gly
   145 150 155 160
   Lys Asn Ile Gln Gly Gly Lys Thr Leu Ser Val Ser Gln Leu Glu Leu
   165 170 175
   Gln Asp Ser Gly Thr Trp Thr Cys Thr Val Leu Gln Asn Gln Lys Lys
   180 185 190
   Val Glu Phe Lys Ile Asp Ile Val Val Leu Ala Phe Gln Lys Ala Ser
   195 200 205
   Ser Ile Val Tyr Lys Lys Glu Gly Glu Gln Val Glu Phe Ser Phe Pro
   210 215 220
   Leu Ala Phe Thr Val Glu Lys Leu Thr Gly Ser Gly Glu Leu Trp Trp
   225 230 235 240
   Gln Ala Glu Arg Ala Ser Ser Ser Lys Ser Trp Ile Thr Phe Asp Leu
   245 250 255
   Lys Asn Lys Glu Val Ser Val Lys Arg Val Thr Gln Asp Pro Lys Leu
   260 265 270
   Gln Met Gly Lys Lys Leu Pro Leu His Leu Thr Leu Pro Gln Ala Leu
   275 280 285
   Pro Gln Tyr Ala Gly Ser Gly Asn Leu Thr Leu Ala Leu Glu Ala Lys
   290 295 300
   Thr Gly Lys Leu His Gln Glu Val Asn Leu Val Val Met Arg Ala Thr
   305 310 315 320
   Gln Leu Gln Lys Asn Leu Thr Cys Glu Val Trp Gly Pro Thr Ser Pro
   325 330 335
   Lys Leu Met Leu Ser Leu Lys Leu Glu Asn Lys Glu Ala Lys Val Ser
   340 345 350
   Lys Arg Glu Lys Ala Val Trp Val Leu Asn Pro Glu Ala Gly Met Trp
   355 360 365
   Gln Cys Leu Leu Ser Asp Ser Gly Gln Val Leu Leu Glu Ser Asn Ile
   370 375 380
   Lys Val Leu Pro Thr Trp Ser Thr Pro Val Gln Pro Met Ala Leu Ile
   385 390 395 400
   Val Leu Gly Gly Val Ala Gly Leu Leu Leu Phe Ile Gly Leu Gly Ile
   405 410 415
   Phe Phe Cys Val Arg Cys Arg His Arg Arg Arg Gln Ala Glu Arg Met
   420 425 430
   Ser Gln Ile Lys Arg Leu Leu Ser Glu Lys Lys Thr Cys Gln Cys Pro
   435 440 445
   His Arg Phe Gln Lys Thr Cys Ser Pro Ile
   450 455
<210> 24
   <211> 856
   <212> PRT
   <213> Human immunodeficiency virus 1
<400> 24
   Met Arg Val Lys Glu Lys Tyr Gln His Leu Trp Arg Trp Gly Trp Arg
   1 5 10 15
   Trp Gly Thr Met Leu Leu Gly Met Leu Met Ile Cys Ser Ala Thr Glu
   20 25 30
   Lys Leu Trp Val Thr Val Tyr Tyr Gly Val Pro Val Trp Lys Glu Ala
   35 40 45
   Thr Thr Thr Leu Phe Cys Ala Ser Asp Ala Lys Ala Tyr Asp Thr Glu
   50 55 60
   Val His Asn Val Trp Ala Thr His Ala Cys Val Pro Thr Asp Pro Asn
   65 70 75 80
   Pro Gln Glu Val Val Leu Val Asn Val Thr Glu Asn Phe Asn Met Trp
   85 90 95
   Lys Asn Asp Met Val Glu Gln Met His Glu Asp Ile Ile Ser Leu Trp
   100 105 110
   Asp Gln Ser Leu Lys Pro Cys Val Lys Leu Thr Pro Leu Cys Val Ser
   115 120 125
   Leu Lys Cys Thr Asp Leu Lys Asn Asp Thr Asn Thr Asn Ser Ser Ser
   130 135 140
   Gly Arg Met Ile Met Glu Lys Gly Glu Ile Lys Asn Cys Ser Phe Asn
   145 150 155 160
   Ile Ser Thr Ser Ile Arg Gly Lys Val Gln Lys Glu Tyr Ala Phe Phe
   165 170 175
   Tyr Lys Leu Asp Ile Ile Pro Ile Asp Asn Asp Thr Thr Ser Tyr Lys
   180 185 190
   Leu Thr Ser Cys Asn Thr Ser Val Ile Thr Gln Ala Cys Pro Lys Val
   195 200 205
   Ser Phe Glu Pro Ile Pro Ile His Tyr Cys Ala Pro Ala Gly Phe Ala
   210 215 220
   Ile Leu Lys Cys Asn Asn Lys Thr Phe Asn Gly Thr Gly Pro Cys Thr
   225 230 235 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Val Asn Phe Thr Asp Asn Ala Lys Thr Ile Ile Val Gln Leu
   275 280 285
   Asn Thr Ser Val Glu Ile Asn Cys Thr Arg Pro Asn Asn Asn Thr Arg
   290 295 300
   Lys Arg Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Ala Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Glu Gly Ser Asn Asn Thr Glu Gly Ser Asp Thr Ile Thr Leu
   405 410 415
   Pro Cys Arg Ile Lys Gln Ile Ile Asn Met Trp Gln Lys Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Tyr Leu Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   His Thr Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Ile
   690 695 700
   Val Asn Arg Val Arg Gln Gly Tyr Ser Pro Leu Ser Phe Gln Thr His
   705 710 715 720
   Leu Pro Thr Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
   740 745 750
   Leu Ala Leu Ile Trp Asp Asp Leu Arg Ser Leu Cys Leu Phe Ser Tyr
   755 760 765
   His Arg Leu Arg Asp Leu Leu Leu Ile Val Thr Arg Ile Val Glu Leu
   770 775 780
   Leu Gly Arg Arg Gly Trp Glu Ala Leu Lys Tyr Trp Trp Asn Leu Leu
   785 790 795 800
   Gln Tyr Trp Ser Gln Glu Leu Lys Asn Ser Ala Val Ser Leu Leu Asn
   805 810 815
   Ala Thr Ala Ile Ala Val Ala Glu Gly Thr Asp Arg Val Ile Glu Val
   820 825 830
   Val Gln Gly Ala Cys Arg Ala Ile Arg His Ile Pro Arg Arg Ile Arg
   835 840 845
   Gln Gly Leu Glu Arg Ile Leu Leu
   850 855
<210> 25
   <211> 483
   <212> PRT
   <213> Human immunodeficiency virus 1
<400> 25
   Ser Ala Thr Glu Lys Leu Trp Val Thr Val Tyr Tyr Gly Val Pro Val
   1 5 10 15
   Trp Lys Glu Ala Thr Thr Thr Leu Phe Cys Ala Ser Asp Ala Lys Ala
   20 25 30
   Tyr Asp Thr Glu Val His Asn Val Trp Ala Thr His Ala Cys Val Pro
   35 40 45
   Thr Asp Pro Asn Pro Gln Glu Val Val Leu Val Asn Val Thr Glu Asn
   50 55 60
   Phe Asn Met Trp Lys Asn Asp Met Val Glu Gln Met His Glu Asp Ile
   65 70 75 80
   Ile Ser Leu Trp Asp Gln Ser Leu Lys Pro Cys Val Lys Leu Thr Pro
   85 90 95
   Leu Cys Val Ser Leu Lys Cys Thr Asp Leu Lys Asn Asp Thr Asn Thr
   100 105 110
   Asn Ser Ser Ser Gly Arg Met Ile Met Glu Lys Gly Glu Ile Lys Asn
   115 120 125
   Cys Ser Phe Asn Ile Ser Thr Ser Ile Arg Gly Lys Val Gln Lys Glu
   130 135 140
   Tyr Ala Phe Phe Tyr Lys Leu Asp Ile Ile Pro Ile Asp Asn Asp Thr
   145 150 155 160
   Thr Ser Tyr Lys Leu Thr Ser Cys Asn Thr Ser Val Ile Thr Gln Ala
   165 170 175
   Cys Pro Lys Val Ser Phe Glu Pro Ile Pro Ile His Tyr Cys Ala Pro
   180 185 190
   Ala Gly Phe Ala Ile Leu Lys Cys Asn Asn Lys Thr Phe Asn Gly Thr
   195 200 205
   Gly Pro Cys Thr Asn Val Ser Thr Val Gln Cys Thr His Gly Ile Arg
   210 215 220
   Pro Val Val Ser Thr Gln Leu Leu Leu Asn Gly Ser Leu Ala Glu Glu
   225 230 235 240
   Glu Val Val Ile Arg Ser Val Asn Phe Thr Asp Asn Ala Lys Thr Ile
   245 250 255
   Ile Val Gln Leu Asn Thr Ser Val Glu Ile Asn Cys Thr Arg Pro Asn
   260 265 270
   Asn Asn Thr Arg Lys Arg Ile Arg Ile Gln Arg Gly Pro Gly Arg Ala
   275 280 285
   Phe Val Thr Ile Gly Lys Ile Gly Asn Met Arg Gln Ala His Cys Asn
   290 295 300
   Ile Ser Arg Ala Lys Trp Asn Asn Thr Leu Lys Gln Ile Ala Ser Lys
   305 310 315 320
   Leu Arg Glu Gln Phe Gly Asn Asn Lys Thr Ile Ile Phe Lys Gln Ser
   325 330 335
   Ser Gly Gly Asp Pro Glu Ile Val Thr His Ser Phe Asn Cys Gly Gly
   340 345 350
   Glu Phe Phe Tyr Cys Asn Ser Thr Gln Leu Phe Asn Ser Thr Trp Phe
   355 360 365
   Asn Ser Thr Trp Ser Thr Glu Gly Ser Asn Asn Thr Glu Gly Ser Asp
   370 375 380
   Thr Ile Thr Leu Pro Cys Arg Ile Lys Gln Ile Ile Asn Met Trp Gln
   385 390 395 400
   Lys Val Gly Lys Ala Met Tyr Ala Pro Pro Ile Ser Gly Gln Ile Arg
   405 410 415
   Cys Ser Ser Asn Ile Thr Gly Leu Leu Leu Thr Arg Asp Gly Gly Asn
   420 425 430
   Ser Asn Asn Glu Ser Glu Ile Phe Arg Pro Gly Gly Gly Asp Met Arg
   435 440 445
   Asp Asn Trp Arg Ser Glu Leu Tyr Lys Tyr Lys Val Val Lys Ile Glu
   450 455 460
   Pro Leu Gly Val Ala Pro Thr Lys Ala Lys Arg Arg Val Val Gln Arg
   465 470 475 480
   Glu Lys Arg
<210> 26
   <211> 3103
   <212> DNA
   <213> Homo sapiens
<400> 26
   ccaggcctag ggtgtggagg agccttgcca tcgggcttcc tgtctctctt catttaagca 60
   cgactctgca gaaggaacaa agcaccctcc ccactgggct cctggttgca gagctccaag 120
   tcctcacaca gatacgcctg tttgagaagc agcgggcaag aaagacgcaa gcccagaggc 180
   cctgccattt ctgtgggctc aggtccctac tggctcaggc ccctgcctcc ctcggcaagg 240
   ccacaatgaa ccggggagtc ccttttaggc acttgcttct ggtgctgcaa ctggcgctcc 300
   tcccagcagc cactcaggga aagaaagtgg tgctgggcaa aaaaggggat acagtggaac 360
   tgacctgtac agcttcccag aagaagagca tacaattcca ctggaaaaac tccaaccaga 420
   taaagattct gggaaatcag ggctccttct taactaaagg tccatccaag ctgaatgatc 480
   gcgctgactc aagaagaagc ctttgggacc aaggaaactt tcccctgatc atcaagaatc 540
   ttaagataga agactcagat acttacatct gtgaagtgga ggaccagaag gaggaggtgc 600
   aattgctagt gttcggattg actgccaact ctgacaccca cctgcttcag gggcagagcc 660
   tgaccctgac cttggagagc ccccctggta gtagcccctc agtgcaatgt aggagtccaa 720
   ggggtaaaaa catacagggg gggaagaccc tctccgtgtc tcagctggag ctccaggata 780
   gtggcacctg gacatgcact gtcttgcaga accagaagaa ggtggagttc aaaatagaca 840
   tcgtggtgct agctttccag aaggcctcca gcatagtcta taagaaagag ggggaacagg 900
   tggagttctc cttcccactc gcctttacag ttgaaaagct gacgggcagt ggcgagctgt 960
   ggtggcaggc ggagagggct tcctcctcca agtcttggat cacctttgac ctgaagaaca 1020
   aggaagtgtc tgtaaaacgg gttacccagg accctaagct ccagatgggc aagaagctcc 1080
   cgctccacct caccctgccc caggccttgc ctcagtatgc tggctctgga aacctcaccc 1140
   tggcccttga agcgaaaaca ggaaagttgc atcaggaagt gaacctggtg gtgatgagag 1200
   ccactcagct ccagaaaaat ttgacctgtg aggtgtgggg acccacctcc cctaagctga 1260
   tgctgagttt gaaactggag aacaaggagg caaaggtctc gaagcgggag aaggcggtgt 1320
   gggtgctgaa ccctgaggcg gggatgtggc agtgtctgct gagtgactcg ggacaggtcc 1380
   tgctggaatc caacatcaag gttctgccca catggtccac cccggtgcag ccaatggccc 1440
   tgattgtgct ggggggcgtc gccggcctcc tgcttttcat tgggctaggc atcttcttct 1500
   gtgtcaggtg ccggcaccga aggcgccaag cagagcggat gtctcagatc aagagactcc 1560
   tcagtgagaa gaagacctgc cagtgtcctc accggtttca gaagacatgt agccccattt 1620
   gaggcacgag gccaggcaga tcccacttgc agcctcccca ggtgtctgcc ccgcgtttcc 1680
   tgcctgcgga ccagatgaat gtagcagatc cccagcctct ggcctcctgt tcgcctcctc 1740
   tacaatttgc cattgtttct cctgggttag gccccggctt cactggttga gtgttgctct 1800
   ctagtttcca gaggcttaat cacaccgtcc tccacgccat ttccttttcc ttcaagccta 1860
   gcccttctct cattatttct ctctgaccct ctccccactg ctcatttgga tcccagggga 1920
   gtgttcaggg ccagccctgg ctggcatgga gggtgaggct gggtgtctgg aagcatggag 1980
   catgggactg ttcttttaca agacaggacc ctgggaccac agagggcagg aacttgcaca 2040
   aaatcacaca gccaagccag tcaaggatgg atgcagatcc agaggtttct ggcagccagt 2100
   acctcctgcc ccatgctgcc cgcttctcac cctatgtggg tgggaccaca gactcacatc 2160
   ctgaccttgc acaaacagcc cctctggaca cagccccatg tacacggcct caagggatgt 2220
   ctcacatcct ctgtctattt gagacttaga aaaatcctac aaggctggca gtgacagaac 2280
   taagatgatc atctccagtt tatagaccag aaccagagct cagagaggct agatgattga 2340
   ttaccaagtg ccggactagc aagtgctgga gtcgggacta acccaggtcc cttgtcccaa 2400
   gttccactgc tgcctcttga atgcagggac aaatgccaca cggctctcac cagtggctag 2460
   tggtgggtac tcaatgtgta cttttgggtt cacagaagca cagcacccat gggaagggtc 2520
   catctcagag aatttacgag cagggatgaa ggcctccctg tctaaaatcc ctccttcatc 2580
   ccccgctggt ggcagaatct gttaccagag gacaaagcct ttggctcttc taatcagagc 2640
   gcaagctggg agcacaggca ctgcaggaga gaatgcccag tgaccagtca ctgaccctgt 2700
   gcagaacctc ctggaagcga gctttgctgg gagagggggt agctagcctg agagggaacc 2760
   ctctaaggga cctcaaaggt gattgtgcca ggctctgcgc ctgccccaca ccctccctta 2820
   ccctcctcca gaccattcag gacacaggga aatcagggtt acaaatcttc ttgatccact 2880
   tctctcagga tcccctctct tcctaccctt cctcaccact tccctcagtc ccaactcctt 2940
   ttccctattt ccttctcctc ctgtctttaa agcctgcctc ttccaggaag acccccctat 3000
   tgctgctggg gctccccatt tgcttacttt gcatttgtgc ccactctcca cccctgctcc 3060
   cctgagctga aataaaaata caataaactt actataaaga tgc 3103
<210> 27
   <211> 7
   <212> PRT
   <213> Artificial Sequence
<220>
   <223> Synthetically generated peptide
<400> 27
   Asp Leu Arg Gln Cys Trp Arg
   1 5
<210> 28
   <211> 12
   <212> PRT
   <213> Artificial Sequence
<220>
   <223> Synthetically generated peptide
<400> 28
   Arg Ile Asn Asn Ile Pro Trp Ser Glu Ala Met Met
   1 5 10
<210> 29
   <211> 99
   <212> PRT
   <213> Homo sapiens
<400> 29
   Lys Lys Val Val Leu Gly Lys Lys Gly Asp Thr Val Glu Leu Thr Cys
   1 5 10 15
   Thr Ala Ser Gln Lys Lys Ser Ile Gln Phe His Trp Lys Asn Ser Asn
   20 25 30
   Gln Ile Lys Ile Leu Gly Asn Gln Gly Ser Phe Leu Thr Lys Gly Pro
   35 40 45
   Ser Lys Leu Asn Asp Arg Ala Asp Ser Arg Arg Ser Leu Trp Asp Gln
   50 55 60
   Gly Asn Phe Pro Leu Ile Ile Lys Asn Leu Lys Ile Glu Asp Ser Asp
   65 70 75 80
   Thr Tyr Ile Cys Glu Val Glu Asp Gln Lys Glu Glu Val Gln Leu Leu
   85 90 95
   Val Phe Gly
<210> 30
   <211> 99
   <212> PRT
   <213> Artificial Sequence
<220>
   <223> Synthetically generated peptide
<220>
   <221> VARIANT
   16
   <222> 60
   <223> Xaa = selenocysteine
<400> 30
   Lys Lys Val Val Leu Gly Lys Lys Gly Asp Thr Val Glu Leu Thr Cys
   1 5 10 15
   Thr Ala Ser Gln Lys Lys Ser Ile Gln Phe His Trp Lys Asn Ser Asn
   20 25 30
   Gln Ile Lys Ile Leu Gly Asn Gln Gly Ser Phe Leu Thr Lys Gly Pro
   35 40 45
   Ser Lys Leu Asn Asp Arg Ala Asp Ser Arg Arg Xaa Leu Trp Asp Gln
   50 55 60
   Val Ala Gly Leu His Leu Ile Lys Asn Leu Lys Ile Glu Asp Ser Asp
   65 70 75 80
   Thr Tyr Ile Cys Glu Val Glu Asp Gln Lys Glu Glu Val Gln Leu Leu
   85 90 95
   Val Phe Gly
<210> 31
   <211> 99
   <212> PRT
   <213> Artificial Sequence
<220>
   <223> Synthetically generated peptide
<220>
   <221> VARIANT
   <222> 60
   <223> Xaa = selenocysteine
<400> 31
   Lys Lys Val Val Leu Gly Lys Lys Gly Asp Thr Val Glu Leu Thr Cys
   1 5 10 15
   Thr Ala Ser Gln Lys Lys Ser Ile Gln Phe His Trp Lys Asn Ser Asn
   20 25 30
   Gln Ile Lys Ile Leu Gly Asn Gln Gly Ser Phe Leu Thr Lys Gly Pro
   35 40 45
   Ser Lys Leu Asn Asp Arg Ala Asp Ser Arg Arg Xaa Leu Trp Asp Gly
   50 55 60
   Leu Gln Val Cys Thr Ile Ile Lys Asn Leu Lys Ile Glu Asp Ser Asp
   65 70 75 80
   Thr Tyr Ile Cys Glu Val Glu Asp Gln Lys Glu Glu Val Gln Leu Leu
   85 90 95
   Val Phe Gly
<210> 32
   <211> 99
   <212> PRT
   <213> Artificial Sequence
<220>
   <223> Synthetically generated peptide
   17
   <220>
   <221> VARIANT
   <222> 60
   <223> Xaa = selenocysteine
<400> 32
   Lys Lys Val Val Leu Gly Lys Lys Gly Asp Thr Val Glu Leu Thr Cys
   1 5 10 15
   Thr Ala Ser Gln Lys Lys Ser Ile Gln Phe His Trp Lys Asn Ser Asn
   20 25 30
   Gln Ile Lys Ile Leu Gly Asn Gln Gly Ser Phe Leu Thr Lys Gly Pro
   35 40 45
   Ser Lys Leu Asn Asp Arg Ala Asp Ser Arg Arg Xaa Leu Trp Asp Gln
   50 55 60
   Val Pro Gly Leu Val Gln Asn Leu Glu Ile Lys Ile Glu Asp Ser Asp
   65 70 75 80
   Thr Tyr Ile Cys Glu Val Glu Asp Gln Lys Glu Glu Val Gln Leu Leu
   85 90 95
   Val Phe Gly
<210> 33
   <211> 100
   <212> PRT
   <213> Artificial Sequence
<220>
   <223> Synthetically generated peptide
<220>
   <221> VARIANT
   <222> 60
   <223> Xaa = selenocysteine
<400> 33
   Lys Lys Val Val Leu Gly Lys Lys Gly Asp Thr Val Glu Leu Thr Cys
   1 5 10 15
   Thr Ala Ser Gln Lys Lys Ser Ile Gln Phe His Trp Lys Asn Ser Asn
   20 25 30
   Gln Ile Lys Ile Leu Gly Asn Gln Gly Ser Phe Leu Thr Lys Gly Pro
   35 40 45
   Ser Lys Leu Asn Asp Arg Ala Asp Ser Arg Arg Xaa His Asp Gln Gly
   50 55 60
   Gly Val Pro Gly Leu Val Arg Asn Leu Lys Ile Lys Ile Glu Asp Ser
   65 70 75 80
   Asp Thr Tyr Ile Cys Glu Val Glu Asp Gln Lys Glu Glu Val Gln Leu
   85 90 95
   Leu Val Phe Gly
   100

## Description

### CROSS-REFERENCE TO RELATED APPLICATIONS

This application claims priority to U.S. Application Serial No, 61/040,128, filed on March 27, 2008.

### TECHNICAL FIELD

The present invention features polypeptides derived from CD4 that bind to the Human Immunodeficiency Virus (HIV) envelope protein gp120. The polypeptides can be used either alone or with heterologous amino acid sequences in prophylactic or therapeutic treatments for HIV infection and Acquired Immune Deficiency Syndrome (AIDS).

### BACKGROUND

HIV-1, also referred to as HTLV-111, LAV or HTLV-III/LAV, is the etiological agent of AIDS and related disorders *(see, e.g.,* Barre-Sinoussi et al., Science 220:868-871, 1983; Gallo et al., Science 224 :500-503, 1984; Levy et al., Science 225:840-842, 1984; Siegal et al., N. Engl. J. Med. 305:1439-1444, 1981). AIDS patients usually experience a long asymptomatic period followed by the progressive degeneration of the immune system and the central nervous system.

Molecular studies of HIV-1 show that it encodes a number of genes (Ratner et al., Nature 313:277-284, 1985; Sanchez-Pescador et al., Science 227:484-492, 1985), including three structural genes that are common to all retroviruses: *gag, pol* and *env.* Nucleotide sequences from viral genomes of other retroviruses, particularly HIV-2 and simian immunodeficiency viruses, SIV (previously referred to as STLV-III), also contain these structural genes (Guyader et al., Nature 326:662-669, 1987).

The envelope protein of HIV-1, HIV-2 and SIV is a glycoprotein of about 160 kDa (gp160). During virus infection of the host cell, gp160 is cleaved by host cell proteases to form gp120 and the integral membrane protein, gp41. The gp41 portion is anchored in the membrane bilayer of the virion, while the gp120 segment protrudes into the surrounding environment. Free gp120 can be released from the surface of virions and infected cells.

Replication of the virus is highly regulated, and both latent and lytic infection of macrophages and the CD4 (cluster of differentiation 4) positive helper subset of T lymphocytes (also known more simply as T cells) occurs in tissue culture *(*Zagury et al.., Science 231:850-853, 1986). When gp 120 binds CD4, the conformation of gp120 changes, subsequently allowing the virus to bind one of either two different chemokine receptors on either the host macrophage or T cell. Following conformational changes in the viral gp41 protein, the outer membrane of HIV fuses with the host cell membrane.

### SUMMARY

This invention is based, in part, on our discovery of certain mutant CD4 polypeptides. More specifically, the invention features polypeptides that include or consist of an amino acid sequence conforming to Formula I: Arg - Arg - Xaa1 - Leu - Xaa2 - Asp - Gln
where Xaa₁ is Cys or Sec and; Xaa₂ is Trp or Phe; with the proviso that the polypeptide is not a wild type CD4 protein or a fragment thereof (*i.e.,* with the proviso that the polypeptide is not a fragment of a wild type CD4 protein). Functionally, the polypeptides bind gp120. While binding can be assessed in a number of ways, the polypeptides are those that compete with an antibody that specifically binds the CD4 binding site of gp120.

In the formula shown above, Xaa₁ is Cys (or "C"; cysteine) or Sec (or "U"; selenocysteine). We may use either the standard three-letter or the standard one-letter code to represent amino acid residues. Thus, the polypeptide can have or can include the sequence RRCLWDQ (SEQ ID NO:2; also referred to in the Examples below as Hel-C); RRULWDQ (SEQ ID NO:3; also referred to in the Examples below as Hel-U); RRULFDQ or RRCLFDQ. Other polypeptides disclosed herein include CRSLWDQ (SEQ ID NO:4); URSLWDQ (SEQ ID NO:5); CRCLWDQ (SEQ ID NO:6); URULWDQ (SEQ ID NO:7); CRULWDQ (SEQ ID NO:8); or URCLWDQ (SEQ ID NO:9). The cognate wild type CD4 sequence, RRSLWDQ (SEQ ID NO:10), is referred to below as Hel-S. In any of these polypeptides, the tryptophan residue at position 5 (W) can also be Phe (F) or Tyr (Y). We may refer to a polypeptide that consists of a sequence conforming to Formula I as a "core polypeptide."

Also described herein are fragments of the core polypeptide. Like the core polypeptides of Formula I, fragments thereof may bind gp120 and may be used to, for example, unmask epitopes, to generate neotopes, or to carry epitopes from heterologous polypeptides and/or non-peptidyl antigens like dinitrobenzene (DNP). Fragments of the core polypeptide may or may not compete with an antibody that specifically binds the CD4 binding site of gp120. For example, the polypeptide RRCLW (SEQ ID NO:11), which is a fragment of the core polypeptide, is useful in the methods described herein. Thus, the specification describes fragments of the core polypeptide that lack one, two, or three residues from the carboxy terminus. Stated in the affirmative, a fragment of a core polypeptide can consist of four, five, or six consecutive amino acid residues conforming to Formula I. As noted, neither the core polypeptide nor fragments thereof have sequences naturally found in wild type CD4 proteins.

For ease of reading, we will not refer repeatedly to "a core polypeptide or a fragment thereof." It is to be understood that where a core polypeptide is useful, an active fragment of the core polypeptide is also useful. Similarly, fragments of a core polypeptide (*e.g.,* polypeptides consisting of the sequence represented by Formula II) can be modified in the same ways a core polypeptide having the sequence represented by Formula I can be modified. For example, as described below, both the core polypeptide and fragments thereof can be flanked by additional CD4 sequence or by heterologous sequence; the side chains of either the core polypeptides or fragments thereof can be modified; and so on,

Further, we tend to use the term "polypeptide" when referring to any chain of amino acid residues, regardless of length, precise sequence or post-translational modification. We may also use the term "peptide" or "protein" when referring to such a chain of amino acid residues. As is common in the art, we may specifically use the term "protein" when referring to a full-length and/or naturally occurring amino acid sequence. For example, when we say that the invention features a polypeptide comprising the amino acid sequence of Formula I, with the proviso that the polypeptide is not a wild type CD4 protein, we mean that the invention excludes full-length, naturally occurring CD4.

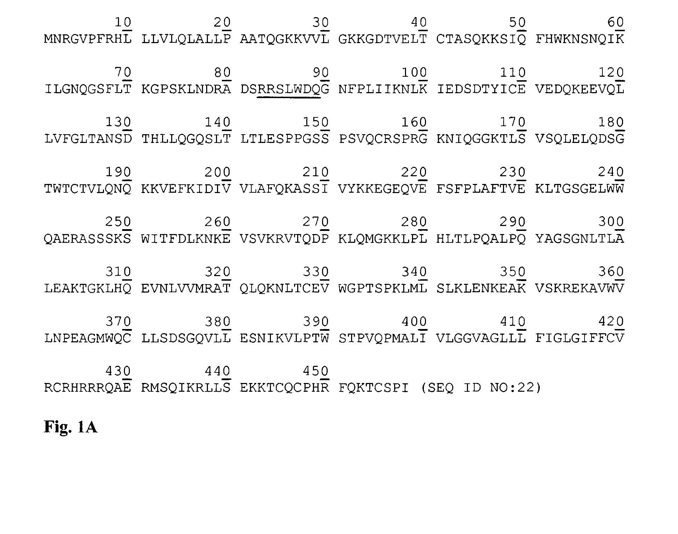

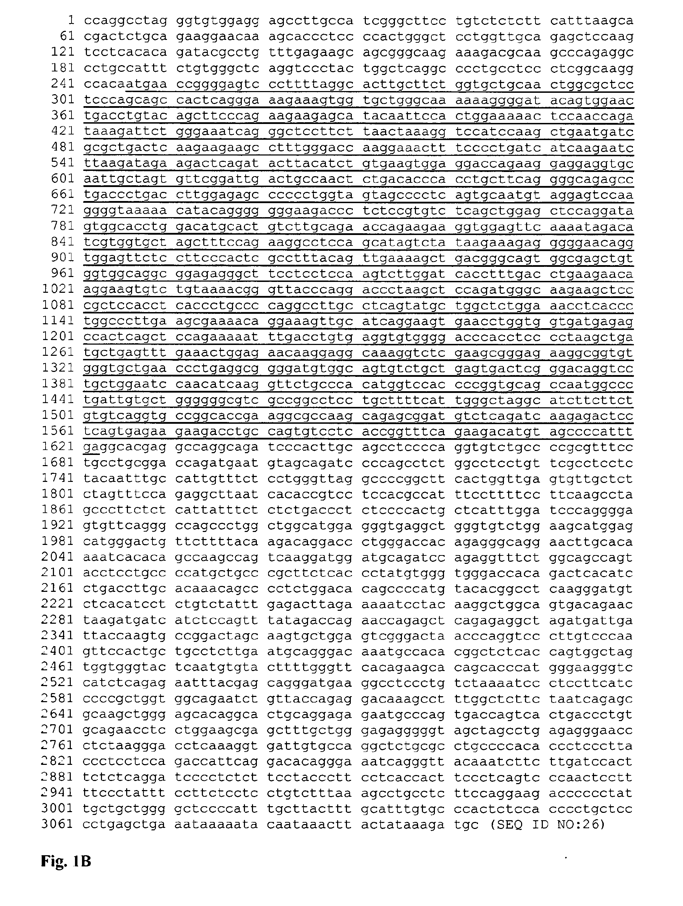

Any of the core polypeptides can be flanked on the amino and/or carboxy terminus by one or more additional residues of the CD4 polypeptide. For example, the polypeptide can include, at its amino terminal, one or more of the flanking residues at position(s) 1-57 of a mature human CD4 (SEQ ID NO:13) or one or more of the flanking residues at position(s) 1-82 of the N-terminal region of a human CD4 protein with its signal sequence, (SEQ ID NO:14). More specifically, a polypeptide of the invention can include or can consist of the sequence SRR(C/U)LWDQ (SEQ ID NO:15; where C/U indicates the inclusion of cysteine or selenocysteine); DSRR(C/U)LWDQ (SEQ ID NO:16); ADSRR(C/U)LWDQ (SEQ ID NO:17); and so on, with amino acid residues being progressively added toward the N-terminal. Alternatively, or in addition, the polypeptide can include, at its carboxy terminal, one or more of the flanking residues at position(s) 65-433 of a mature human CD4 (SEQ ID NO:18; corresponding to residues 90-458 when the signal sequence is present, as shown in Fig. 1). More specifically, the polypepide can include or can consist of the sequence RR(C/U)LWDQG (SEQ ID NO:19); RR(C/U)LWDQGN (SEQ ID NO:20); RR(C/U)LWDQGNF (SEQ ID NO:21); and so on, with amino acid residues being progressively added toward the C-terminal. In one embodiment, the polypeptide can include or can consist of a wild type CD4 polypeptide with a mutation at position 60 and/or position 62 of the mature CD4 polypeptide *(i.e.,* the polypeptide can include or can consist of the sequence of a CD4 polypeptide, with or without a signal sequence, except for the inclusion of a core polypeptide where CD4 wild type sequence would normally reside).

For ease of reading, we will not continue to repeat the phrase "can include or can consist of." It is to be understood that the polypeptides of the invention can consist of the sequence explicitly identified (for example, the core polypeptides consist of a sequence conforming to Formula I) or can include additional amino acid residues, and/or participate in the formation of larger molecular entities, such as complexes (*e.g.,* a core polypeptide or a fragment thereof can be fused to additional CD4 sequence or complexed to another polypeptide, such as gp120).

Instead of being flanked by additional CD4 sequence, any of the core polypeptides can be flanked on either the amino and/or carboxy terminus by one or more residues of a heterologous polypeptide (*i.e.,* a polypeptide other than CD4). For example, a core polypeptide can be flanked on either the N- or C-terminus with 1 to about 1,000 amino acid residues (*e.g.,* about 5, 10, 15, 20, 25; 50, 100; 150, 200, 250, 300, 400, 500, 600, 700, 800, or 900 amino acid residues) of a heterologous polypeptide. The heterologous sequence can be that of a scorpion toxin (scyllatoxin) scaffold (Martin et al., Nature Biotechnology 21:71-76, 2003; Huang et al., Structure 13:755-768, 2005) or other heterologous sequence that provides a structural scaffold that enhances binding of the core polypeptide to gp120 The heterologous sequence can also be, or can include, a B cell or a T cell epitope.

Alternatively or in addition, a core polypeptide can be flanked by a non-peptidyl antigen (*e.g.,* DNP). The polypeptides of the invention can be bound to non-peptidyl antigens by methods known in the art. For example, DNP can be bound to the present polypeptides by the methods described in Shokat and Schultz (J. Am. Chem. Soc. 113:1861-1862, 1991).

The heterologous sequence at one or more ends of the core polypeptide can also be used to increase the circulating half life of the core polypeptide. For example, the heterologous amino acid sequence can be that of albumin or an immunoglobulin (*e.g.,* the Fc region of an immunoglobulin). The heterologous sequence can be that of a full length protein or it may be a fragment thereof of sufficient size to confer substantially the same benefit or achieve substantially the same result as the full length protein. For example, the heterologous sequence can represent at least or about 50%, 60%, 70%, 80%, 90% or 95% of a full length protein. Where the heterologous sequence is attached to the core polypeptide by a peptide bond, we may refer to the resulting polypeptide as a fusion protein.

The side chain of one or more amino acid residues within either the core polypeptide or extended fusion proteins can be modified by a number of methods (*e.g.,* glycosylation) and including click chemistry conjugation methods which involve 1,3-bipolar cycloaddition of azides and terminal alkynes. One or more of the amino acid residues can be in the D-form rather than the L-form.

The present polypeptides can also be cyclized by the formation of intramolecular bonds (*e.g.,* intramolecular disulphide and/or diselenide bonds and/or seleno-sulphide bonds). Due to the presence of cysteine and/or selenocysteine, the core polypeptides may dimerize with one another. Accordingly, monomers, dimmers, compositions containing them, and compositions containing a mixture of polypeptides, some in monomeric form and some in dimeric form, are within the scope of the present invention.

In other embodiments, a polypeptide that includes or consists of a core polypeptide can be associated with gyp120 or a portion thereof in a non-linear manner (*i.e.,* a polypeptide of the invention can be complexed with gp120). For example, a polypeptide consisting of or comprising a core polypeptide can be associated with all or a portion of gp120 by a disulphide, trisulphide, or seleno-sulphide bond; by a crosslink produced, for example, with a fixative; or by non-covalent interaction (*e.g.,* by an electrostatic interaction, van der Waals force, or a hydrogen bond). The resulting complexes are within the scope of the invention, as are antibodies that specifically bind the complexes.

While the present polypeptides, fusion proteins, and polypeptide complexes are not limited to those that function by any particular mechanism, we expect that lengthening a core polypeptide or complexing it with another polypeptide may create an entity that sterically hinders the interaction between gp120 and CD4 *in vivo* and, thereby, inhibits HIV entry into a host cell. Where the core polypeptide is extended to include B cell or T cell epitopes, or complexed with such epitopes, the resulting fusion proteins or complexes may be used as carriers to deliver antigenic or immunogenic epitopes to the surface of HIV where they could serve as targets for antibodies (*e.g.,* neutralizing antibodies). Where a core polypeptide is complexed with gp120 (or a portion thereof), the result may be the unmasking of an epitope or epitopes on the associated gp120 or the generation of a new epitope or neotope. *In vivo,* the core polypeptides may create neotopes upon binding with gp120.

Where an immune response is elicited or enhanced by the present polypeptides, the response can include an antibody-mediated immune response and/or a cell-mediated immune response. For example, cytotoxic T cells (CTLs) can be generated, resulting in a cytolytic immune response. Accordingly, and as noted above, the heterologous polypeptide fused to or complexed with a core polypeptide can include the sequence of one or more B cell and/or T cell epitopes.

The core polypeptides and extended polypeptides can also be associated with (*e.g.,* bound to) non-protein chemical compounds. For example, the polypeptides can be bound to a large molecule such as polyethylene glycol (PEG) or to a non-peptidyl antigen such as DNP. The polypeptides can also be bound to a B complex vitamin or a form of vitamin E.

The invention also features nucleic acid molecules that include or consist of a sequence encoding the core polypeptides and fusion proteins described herein, as well as expression vectors containing these nucleic acid molecules (*e.g.,* a plasmid, cosmid, or viral vector). In another aspect, the invention features cells (*e.g.,* a bacterial cell such as *E. coli*) that include the nucleic acid molecules or vectors.

The present polypeptides, nucleic acids, vectors, and cells can be formulated, either alone, in combination with one another, or in combination with a physiologically acceptable carrier, as a pharmaceutical composition. While these compositions are described further below, we note here that they can be formulated for nasal, sublingual, subcutaneous, intravenous or oral administration. The pharmaceutical compositions can also include a simple mixture of a core polypeptide and a gp120 protein or an active fragment thereof.

Other compositions of the present invention include compositions containing a core polypeptide, a fusion protein, or a complexed polypeptide in a form that is suitable for mixing with whole blood or a fraction thereof (*e.g.,* in a lyophilized form or in a solution). Yet other compositions are blood samples including a polypeptide described herein and kits that include a polypeptide, nucleic acid, expression vector or cell as described herein and instructional materials for use. The kits can include paraphernalia such as a syringe, and can be used in the process of making recombinant polypeptides, preparing blood samples for transfusion (or any blood-based product for use), or administering a polypeptide to a patient.

With respect to treatment, the polypeptides and pharmaceutical compositions described herein can be used in methods of treating a subject who has been, or who is at risk of being, exposed to a human immunodeficiency virus. The patient can have AIDS, and can be a human subject. The compositions can be administered for a time and under conditions (*e.g.,* in an effective amount) such that the recipient experiences an improvement in a sign or symptom associated with AIDS or has a reduced risk or likelihood of contracting AIDS. Risk or likelihood may be determined based on statistical probabilities. Formulations and routes of administration are described further below.

Because of the binding properties of the polypeptides described herein, they can be used to detect or to purify gp120, other HIV envelope proteins or protein complexes containing gp120, or viruses expressing gp120. The present mutant CD4 polypeptides can be incorporated in place of anti-gp 120 antibodies or wild type CD4 (or any other CD4 polypeptide) in binding or purification assays, including those presently known in the art of HIV detection and purification. The methods that result in gp 120 detection can be carried out on any sample of interest. These include samples of tissue or bodily fluids obtained from a patient, and the outcome (presence or absence of gp120) can be used to diagnose or to aid in the diagnosis of a patient who has been infected with a virus that expresses gp120. The detection methods can also be used to detect a gp-120 expressing virus in a sample of blood given, for example, to a blood bank or in an organ or tissue under consideration for transplantation. To facilitate detection, the CD4 polypeptide can be tagged with a label that generates a signal *(e.g.,* a visual signal (*e.g.,* a colorimetric change or fluorescence) or a radioactive signal). For example, the polypeptide can include a histidine tag, another metal chelator or epitope tag, a radioisotope, or the like. The methods can be carried out in solution or with the CD4 polypeptide bound (*e.g.,* via a linker) to a solid substrate such as a column or well.

The purification methods similarly require exposure of a CD4 polypeptide as described herein to a sample that contains or is believed to contain gp120 or a gp120-expressing virus. As with the detection methods, the provided sample and the CD4 polypeptide can be placed under conditions in which binding should occur so long as both binding partners are present. As with the detection methods, the CD4 polypeptide can be detectably labeled and the reaction can be carried out in solution or with the CD4 polypeptide bound to a substrate. Whether used for detection or purification, the present polypeptides may be superior to gp120-binding partners currently available, as the present polypeptides may be able to detect gp120 that has naturally mutated.

The details of one or more embodiments of the invention are further set forth in the accompanying drawings and the description below. Other features, objects, and advantages of the invention will be apparent from the description, the drawings, and the claims.

### DESCRIPTION OF DRAWINGS

Fig. 1A (SEQ ID NO:22) is the primary amino acid sequence of wildtype unprocessed human CD4 (GenBank Accession No. P01730) with the amino acid sequence from the 83^{rd} to the 89^{th} residues underlined. A mature form of the polypeptide extends from residue 26 to residue 433.
Fig. 1B (SEQ ID NO:26) is the cDNA sequence of human CD4 (GenBank Accession No. NM_000616, GeneID: 920), with the coding sequence spanning nucleotides 246-1622 underlined.
Fig. 2 (SEQ ID NO:23) is the primary amino acid sequence of a mutant of unprocessed human CD4 with a serine to cysteine substitution at the 85^{th} residue (shown in bold, with a core polypeptide sequence underlined).
Fig. 3 is a Table depicting representative core polypeptides (SEQ ID NOs 2, 3, 6, 7, 8, and 9).
Fig. 4 (SEQ ID NO:24) is an exemplary primary amino acid sequence for the HIV-1 gp160 polypeptide (Genbank Accession No. NP_057856.1) with the amino acid sequence of gp120 underlined.
Fig. 5 (SEQ ID NO:25) is an exemplary primary amino acid sequence for the HIV-1 gp120 polypeptide (Genbank Accession No. NP_579894.2).
Fig. 6 is an alignment of a portion of wild type CD4 (the N-terminal 99 amino acid residues of the mature form; SEQ ID NO:29) and corresponding portions of four polypeptides including amino acid changes incurred as a result of the introduction of SECIS elements into the 2dCD4 ORFs downstream of the Sec60-encoding stop-codon (SEQ ID NOs. 30-33, respectively).

### DETAILED DESCRIPTION

The first step of HIV-1 entry into host cells involves an interaction between the viral envelope protein (Env) and the host cell CD4 receptor. Functionally active (fusogenic) Env exists on the surface of virions as a trimer composed of 3 heterodimeric gp120/gp41 complexes. The latter are established through non-covalent association of monomeric gp120 with membrane-embedded gp41, such that gp120 is presented on the exterior of the viral lipid membrane for interaction with CD4. As the primary contact point of the cellular host receptor proteins and the foremost target of the humoral immune response induced during viral infection, much research has focused on the development of both Env-directed therapeutic compounds and Env-based immunogens designed to elicit broadly neutralizing anti-HIV antibodies. To a large extent, these efforts have been frustrated by significant sequence heterogeneity now known to exist among Envs, even from viruses that are phylogenetically closely related. This heterogeneity limits the desired cross reactivity of anti-Env therapies and antibodies. Furthermore, Env is extensively glycosylated and conformationally flexible, which compound the problems of Env ligand accessibility and reactivity, respectively. Nevertheless, a few monoclonal antibodies that bind Env and have broad viral neutralization activity have been identified. One such antibody is IgG b12, a monoclonal anti-gp120 antibody that neutralizes a large variety of primary HIV-1 isolates and protects Rhesus macaques against vaginal challenge with pathogenic SHIVs.

We have examined structural models of the CD4-gp120 complex and evaluated the potential biochemical effects of several conservative mutations of CD4 gp120-interacting residues. Our aim was to identify amino acid changes in the CD4 sequence that could be incorporated into the design of CD4-mimetic peptides (*e.g.,* what we now refer to as the core polypeptides) to enhance the favorability of interactions with gp120 at the CD4 binding site. One particular CD4 mutation we studied was S60C, which resulted in a striking theoretical enhancement of the interaction with gp120. Regarding positional equivalence, the serine residue in question appears at position 60 in the mature or processed CD4 polypeptide and at position 85 of the polypeptide when its signal sequence is included, as shown in Fig. 1. Mutations within the core polypeptide may be referred to with respect to either the processed or unprocessed sequence *(e.g.,* S60C and S60U are the equivalents of S85C and S85U, respectively).

When modeled on a crystal structure of CD4 in complex with gp120 (PDB ID 1G9M), one rotamer of the S60C mutation formed a novel disulphide bond with 126C at the base of the gp120 V1/V2 loop, which corresponded with (and was presumably responsible for) a large negative rotamer score of -20. We proposed, therefore, that the stereochemically conservative S60C CD4 mutation might result in negligible structural deviation from the wild-type sequence, and simultaneously promote a highly favourable interaction based on covalent bond formation between the mutant sequence and the target gp120.

*CD4 polypeptides:* In the Examples below, we present data showing that the linear CD4-mimetic peptide Hel-C (RRCLWDQ (SEQ ID NO:2)), corresponding to R58 to Q64 of a mature CD4 (but with a S60C mutation), is capable of binding recombinant gp120 at a position that overlaps with the IgG b12 epitope, and that this binding is associated with the formation of disulphide bonds or even trisulphide bonds. We propose that the capacity of appropriately targeted Cys-containing or selenocysteine containing CD4-mimetic peptides to bind at the CD4 binding site (CD4bs), presumably through establishment of a disulphide bond at the base of the V1/V2 loop, has several profound applications for both prophylactic and therapeutic treatment of HIV and AIDS.

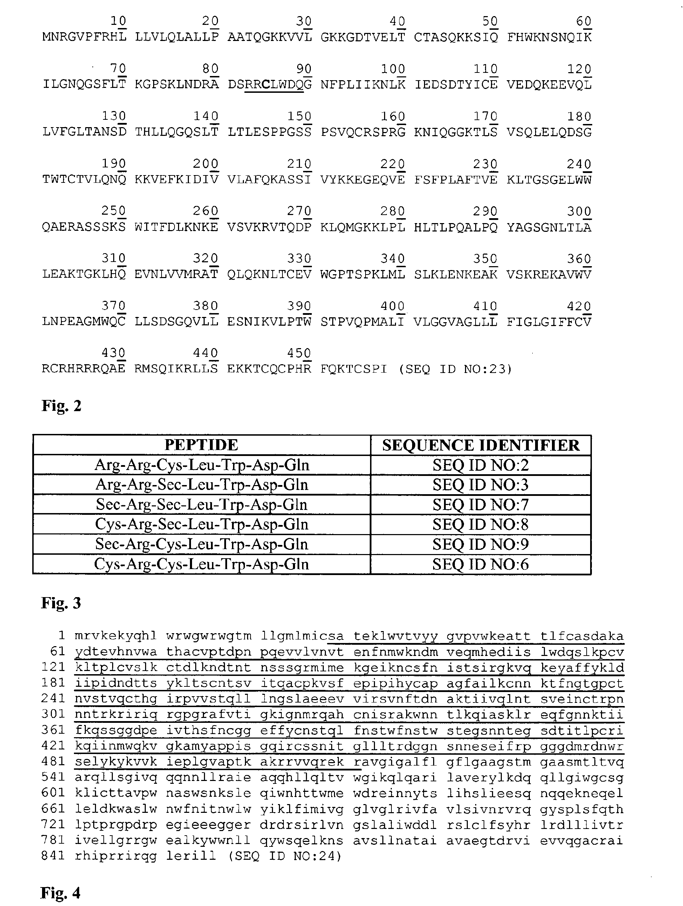

Accordingly, the present polypeptides include mutants of CD4 that bind gp120. The polypeptides can constitute (a) a full-length CD4 that includes at least one mutation, including any of the mutations allowed by Formula I within the core polypeptide, or (b) a fragment of a CD4 polypeptide including the core-polypeptide (*e*.*g*., an 8-, 9-, 10-, 20-, 30-, 40-, 50-, 60-, 70-, 80-, 90-, 100-, 110-, 120-, 130-, 140-, 150-, 175-, 200-, 225-, 250-, 275-, 300-, or 350-amino acid residue fragment of CD4 including the core polypeptide). The polypeptide can also consist of a 7-amino acid polypeptide conforming to the formula in claim 1 (*i.e.,* the polypeptide can consist of a core polypeptide, as shown, for example, in Fig. 3). The full-length CD4 mutant polypeptide, a fragment thereof, the core polypeptide or a fragment thereof can be fused to a heterologous sequence (*e.g.,* a gp120 protein or a fragment thereof).

As noted, the invention features polypeptides that include or consist of an amino acid sequence conforming to Formula I: Arg - Arg - Xaa1 - Leu - Xaa2 - Asp - Gln
where Xaa₁ is Cys or Sec; Xaa₂ is Trp or Phe; with the proviso that the polypeptide is not a wild type CD4 protein or a fragment thereof. Functionally, the polypeptides bind gp120. While binding can be assessed in a number of ways, the polypeptides include those that compete with an antibody that specifically binds the CD4 binding site of gp120 (such as b12). As noted above, fragments of the core polypeptide may not be competitive with an antibody that binds the CD4 binding site of gp120.

In the formulas shown above, Xaa₁ is Cys or Sec. Thus, the present polypeptides can have or can include the sequence RRCLWDQ (SEQ ID NO:2); RRULWDQ (SEQ ID NO:3); RRULFDQ and RRCLFDQ. Other polypeptides disclosed herein include CRSLWDQ (SEQ ID NO:4); URSLWDQ (SEQ ID NO:5); CRCLWDQ (SEQ ID NO:6); URULWDQ (SEQ ID NO:7); CRULWDQ (SEQ ID NO:8); or URCLWDQ (SEQ ID NO:9) or can be a fragment of a core polypeptide according to Formula II.. In any of these polypeptides, the tryptophan residue at position 5 (W) can also be Phe (F) or Tyr (Y).

The core polypeptide described above can be extended by the inclusion of additional CD4 sequence. The amino acid sequence of unprocessed CD4 is 458 amino acids residues in length (SEQ ID NO:22 and Fig. 1). Provided that the residues are numbered consecutively from one to 458, residues one to 25 form a signal sequence that is cleaved resulting in the mature protein, also known as T cell glycoprotein CD4, which consists of residues 26 to 458. Residues 26 to 396 constitute a putative extracellular domain; residues 397 to 418 constitute a putative transmembrane domain; and residues 419 to 458 constitute a putative cytoplasmic domain. Other features are as follows. Residues 26 to 125, 126 to 243, 204-317, and 318 to 374 form immunoglobulin-like domains D1, D2, D3 and D4 and the first mediates CD4 binding to gp120. The polypeptides of the present invention retain sequences that are required for binding with gyp120 (for example, the D1 domain). Accordingly, the present polypeptides can include some (*e.g*., at least 5%, 10%, 15%, or 20%) or essentially all of the sequence on either the N-terminal and/or the C-terminal side of the core polypeptides that constitute the sequence of D1. However, it may be desirable to remove or excise part of D1 or other parts of the CD4 sequence for various reasons (to enable large-scale protein production, for example). For example, the 25 amino acid signal sequence may be deleted and/or replaced for the purposes of Recombinant protein production. In another example, the transmembrane and cytoplasmic domains may be deleted to make a soluble form of the protein.

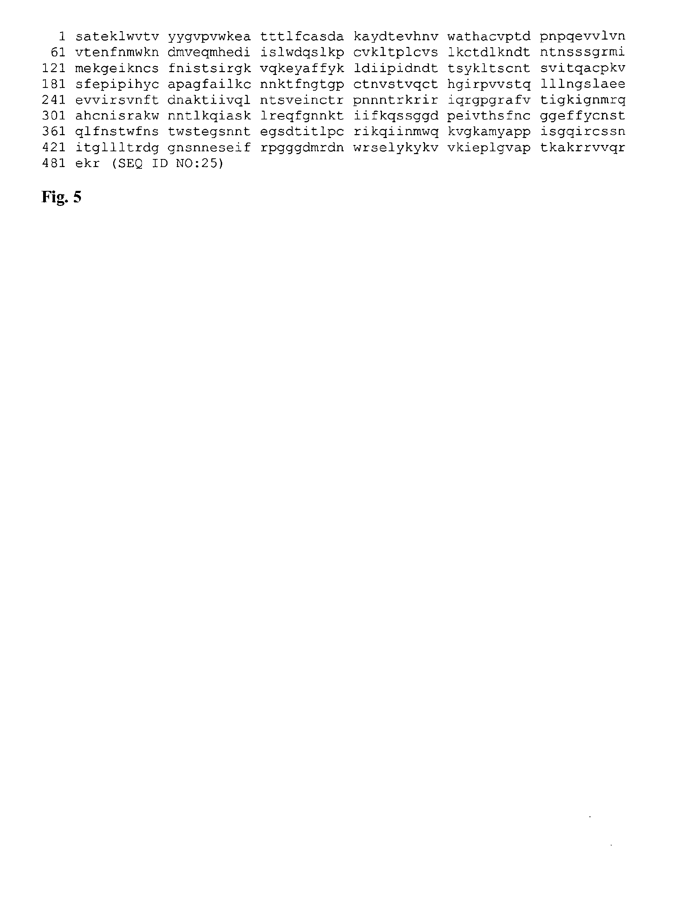

The present polypeptides, including the core polypeptides, and extended variants of these sequences (*e.g.,* variants in which a core polypeptide or a fragment thereof is fused by a peptide bond to B cell and/or T cell epitope sequences) can be used together with a gp120 sequence (*e.g*., as shown in Fig. 4 or 5). More specifically, the present polypeptides can be complexed with gp120 or a portion thereof in a non-linear manner (*i.e.,* joined by virtue of a bond other than a peptide bond such that the entities within the resulting complex are associated other than by virtue of a single contiguous sequence). In that event, the polypeptide and the heterologous sequence (*e.g*., gp120) can be joined by a disulphide (or trisulphide) bond, another covalent bond, or a non-covalent bond. The present CD4 polypeptides can also be used together with a gp120 sequence by virtue of being contained within the same preparation (*e.g.,* mixed within the same solution). The uncleaved precursor of gp120 (Env or gp160) may be used so long as the gp120 polypeptide includes a CD4 binding region.

The gp120 can be obtained from any HIV clade or isolate. For example, the gp120 sequences used in the context of the present invention (*i.e.,* those complexed with the present polypeptides) can be the homologue of a gp120 protein from any clade or from immunodeficiency viruses infecting other species (*e.g.,* FIV or SIV). Variants of gp120 proteins can be used and can be functionally equivalent to a wild type and/or full length gp120 protein. "Functionally equivalent" variants will have sufficient biological activity relative to that of their wild type counterpart to be clinically or medically useful. The biological activity includes binding to CD4 and immunogenicity. The variants of gp120 can vary by virtue of a deletion or mutation (*e.g.,* a conservative substitution) of one or more residues at, for example, a non-essential position. The CD4 portion of the polypeptide can be similarly modified. In one embodiment, the gp120 polypeptide and/or the CD4 polypeptide can include an amino acid sequence that is at least or about 60% identical to a corresponding wild type sequence or a fragment thereof. Preferably, the polypeptide is at least 65%, 70%, 75%, 80%, 85%, 90%, 95%, 98%, 99% or more identical to a corresponding wild type sequence (e.g., to SEQ ID NO:22 or residues 26-433 of SEQ ID NO:22).

gp120 polypeptides useful as described herein are those derived from a gp120 region of the Env polypeptide. Preferably, the gp120 polypeptide is derived from HIV Env. The primary amino acid sequence of gp120 is approximately 511 amino acids long, with a polypeptide core of about 60,000 Daltons. The polypeptide is extensively modified by N-linked glycosylation to increase the apparent molecular weight of the molecule to 120,000 Daltons. The amino acid sequence of gp120 contains five relatively conserved domains interspersed with five hypervariable domains. The positions of the 18 cysteine residues in the gp120 primary sequence of the HIV-1HxB-2 (hereinafter "HXB-2") strain, and the positions of 13 of the approximately 24 N-linked glycosylation sites in the gp120 sequence are common to most, if not all, gp120 sequences. The hypervariable domains contain extensive amino acid substitutions, insertions and deletions. Despite this variation, most, if not all, gp120 sequences preserve the virus's ability to bind to the viral receptor CD4. A "gp120 polypeptide" includes both single subunits and/or multimers.

Env polypeptides (*e.g.,* gp120, gyp140 and gp160) include a "bridging sheet" comprised of 4 anti-parallel β -strands (β-2, β-3, β-20 and β-21) that form a β-sheet.

Extruding from one pair of the β-strands (β-2 and β-3) are two loops, V1 and V2. The β-2 sheet occurs at approximately amino acid residue 119 (Cys) to amino acid residue 123 (Thr) while β-3 occurs at approximately amino acid residue 199 (Ser) to amino acid residue 201 (Ile), relative to amino acid sequences of HIV-1 strain HXB-2. The "V1/V2 region" occurs at approximately amino acid positions 126 (Cys) to residue 196 (Cys), relative to HXB-2. (see, *e.g.,* Wyatt et al. J. Virol. 69: 5723-5733, 1995; Stamatatos et al., J. Virol. 72: 7840-7845, 1998). Extruding from the second pair of β-strands (β-20 and β-21) is a small-loop structure, also referred to herein as "the bridging sheet small loop." In HXB-2, β-20 extends from about amino acid residue 422 (Gin) to amino acid residue 426 (Met) while β-21 extends from about amino acid residue 430 (Val) to amino acid residue 435 (Tyr). In variant SF162, the Met-426 is anArg (R) residue. The "small loop" extends from about amino acid residue 427 (Trp) through 429 (Lys), relative to HXB-2. Alignment of the amino acid sequences of Env polypeptide gp160 of any HIV variant can be determined relative to other variants, such as HXB-2, as described for example, in WO 00/39303.

The gp120 polypeptides that can be used with (*e.g.,* complexed with or mixed with) the core polypeptides, or extended variants of either, are not limited to those having the exact sequence of any particular polypeptide described herein. Indeed, the HIV genome is in a state of constant flux and contains several variable domains that exhibit relatively high degrees of variability between isolates. It is readily apparent that the terms encompass Env (*e.g.,* gp120) polypeptides from any of the identified HIV isolates, as well as newly identified isolates, and subtypes of these isolates. Descriptions of structural features are given herein with reference to HXB-2. One of ordinary skill in the art in view of the teachings of the present disclosure and the art can determine corresponding regions in other HIV variants (*e.g.,* isolates HIVIIb, HIVSF2, HIV-1SF162, HIV-1SF170, HIVLAV, HIVLAI, HIVMN, HTV-1cM235, HIV-1US4, other HIV-1 strains from divers subtypes (*e.g*., subtypes, A through G, and O), HIV-2 strains and diverse subtypes (*e*.*g*., HIV-2Uc1 and HIV-2UC2), and simian immunodeficiency virus (SIV). (See, *e.g.,* Virology, 3rd Edition (W. K Joklik ed. 1988); Fundamental Virology, 2nd Edition (B. N. Fields and D. M. Knipe, eds. 1991); Virology, 3rd Edition (Fields, BN, DM Knipe, PM Howley, Editors, 1996, Lippincott- Raven, Philadelphia, PA; for a description of these and other related viruses, using for example, sequence comparison programs (*e.g.,* BLAST and others described therein) or identification and alignment of structural features (*e.g.,* a program such as the "ALB" program described therein that can identify (3-sheet regions)). The actual amino acid sequences of the modified Env polypeptides can be based on any HIV variant.

As noted above, the polypeptides *(e.g.,* gp120) included within the present complexes can be modified relative to the native sequence. For example, the heterologous polypeptides can include terminal or internal deletions, additions and/or substitutions. These modifications may be deliberate, as through site-directed mutagenesis, or may be accidental, such as through naturally occurring mutational events. Thus, for example, if the Env polypeptide is to be used in June 2014 vaccine compositions, the modifications must be such that immunological activity (*i.e.,* the ability to elicit an antibody response to the Env polypeptide or the complex) is not lost.

Thus, a "modified Env polypeptide" can be an Env polypeptide (*e.g.,* gp120 as described - above), which has been complexed to a CD4 polypeptide of this invention that, optionally, has a modified sequence (*e.g.,* a mutation in the in the variable regions V1 and V2). Thy Env polypeptide may be monomeric or oligomeric.

Complexing the present polypeptides and Env (*e.g.,* gp120) can result in exposure of epitopes in or near the CD4 binding site, while allowing correct folding (*e.g.,* correct geometry) of the Env polypeptide. Additionally, modifications (*e.g.,* truncations) to the variable loop regions (V1, V2, V3, V4 and/or V5) may also be made.

Methods of introducing mutations for preparing polypeptides that are functionally equivalent to another polypeptide are well known to one of ordinary skill in the art. For example, one may use site-directed mutagenesis (Hashimoto-Goto et al., Gene 152:271-275, 1995; Zoller et al., Methods Enzymol. 100:468-500, 1983; Kramer et al., Nucl. Acids Res., 12:9441-9456, 1984; Kramer et al., Methods. Enzymol., 154:350-367, 1987; Kunkel, Proc. Natl. Acad. Sci. USA, 82:488-492, 1985; Kunkel, Methods Enzymol., 85:2763-2766, 1988) and such in order to introduce an appropriate mutation into the (amino acid sequence of CD4 and/or gp120 and prepare a protein that is functionally equivalent to the protein at hand. Mutations of amino acids may occur in nature as well.

A fusion protein containing the polypeptides described herein is an example of an extended protein in which one or more amino acid residues have been added to the amino acid sequence of a core polypeptide. A fusion protein is made by fusing a core polypeptide with another peptide (or peptides) or protein (or proteins) and are included in the present invention. A fusion protein can be prepared by ligating a DNA encoding the polypeptides of the present invention with a DNA encoding another peptide(s) or protein(s) in frame, introducing the ligated DNA into an expression vector, and expressing the fusion gene in a host. Methods known by one skilled in the art can be used for preparing such a fusion gene. There is no restriction as to the other peptide(s) or protein(s) that is (are) fused to the protein of this invention.

Other amino acid sequences to be fused with a polypeptide of the present invention are known peptides, for example, FLAG (Hopp et al., Biotechnology 6:1204-1210, 1988), 6x His constituting six histidine (His) residues, 10x His, Influenza agglutinin (HA), human c-myc fragment, VSV-GP fragment, p18HIV fragment, T7-tag, HSV-tag, E-tag, SV40T antigen fragment, lck tag, tubulin fragment, B-tag, Protein C fragment, and so on. Other examples of peptides to be fused with the protein of the present invention are the glutathione-S-transferase (GST), influenza agglutinin (HA), immunoglobulin constant region, β-galactosidase, maltose-binding protein (MBP), and the like.

Fusion proteins can be prepared by fusing commercially available DNA encoding these peptides or proteins with DNA encoding a protein of the present invention and expressing the fused DNA prepared.

The hybridization technique (Sambrook et al., Molecular Cloning 2nd ed. 9.47-9.58, Cold Spring Harbor Laboratory Press, 1989) is well known to one skilled in the art as an alternative method for preparing a protein functionally equivalent to a certain protein. More specifically, one can utilize the general procedure to obtain a protein functionally equivalent to the polypeptides described herein by isolating DNA having a high homology with the whole or part of the DNA encoding the polypeptides described herein. Thus, the present invention includes such proteins, that are encoded by DNAs that hybridize with a DNA consisting of a DNA encoding polypeptides described herein or part thereof and that are functionally equivalent to polypeptides described herein. For instance, homologues of human CD4 in other mammals (such as those of monkey, mouse, rabbit, and bovine) are included. In order to isolate a cDNA with high homology to a DNA encoding a human CD4 from animals, it is preferable to use tissues such as heart, placenta, and testis.

Stringent hybridization conditions for isolating DNA encoding functionally equivalent proteins of polypeptides described herein can be suitably selected by one skilled in the art, and for example, low-stringent conditions can be given. Low-stringent conditions are, for example, 42°C, 2x SSC, and 0.1% SDS, and preferably, 50°C, 2x SSC, and 0.1% SDS. Highly stringent conditions are more preferable and include, for example, 65°C, 2x SSC, and 0.1% SDS. Under these conditions, the higher the temperature, the higher the homology of the obtained DNA will be. However, several factors other than temperature, such as salt concentration, can influence the stringency of hybridization and one skilled in the art can suitably select the factors to accomplish a similar stringency.

In place of hybridization, the gene amplification method, for example, the polymerase chain reaction (PCR) method can be utilized to isolate the object DNA using primers synthesized based on the sequence information of the DNA encoding the polypeptides described herein.

Proteins that are functionally equivalent to polypeptides described herein, encoded by DNA isolated through the above hybridization technique or by the gene amplification technique, normally have a high homology to the amino acid sequence of the test polypeptide (for example, either of the polypeptides described herein).

The amino acid sequence, molecular weight, isoelectric point, the presence or absence of the sugar chain, and the form of a protein of the present invention may differ according to the producing cells, host, or purification method described below. However, so long as the obtained protein has an equivalent function to a protein of the present invention (for example, SEQ ID NO:2), it is included in the present invention. For example, if a protein of the present invention is expressed in prokaryotic cells, such as *E. coli,* a methionine residue is added at the N-terminus of the amino acid sequence of the expressed protein. If a protein of the present invention is expressed in eukaryotic cells, such as mammalian cells, the N-terminal signal sequence is removed. Such proteins are also included in the proteins of the present invention.

A protein of the present invention can be prepared by methods known to one skilled in the art, as a recombinant protein, and also as a natural protein. A recombinant protein can be prepared by inserting a DNA encoding a protein of the present invention (for example, the DNA comprising the nucleotide sequence of SEQ ID NO:2) into a suitable expression vector, introducing the vector into a suitable host cell, and collecting the protein from the resulting transformant. After obtaining the extract, recombinant protein can be purified and prepared by subjecting to chromatography, such as ion exchange chromatography, reverse phase chromatography, gel filtration, and such, or affinity chromatography, to which antibodies against the protein of the invention are immobilized, or combining one or more of these columns.

Further, when a protein of the present invention is expressed within host cells (for example, animal cells and *E. coli*), as a fusion protein with glutathione-S-transferase protein or as a recombinant protein supplemented with multiple histidines, the expressed recombinant protein can be purified using a glutathione column or nickel column.

After purifying the fusion protein, it is also possible to exclude regions other than the objective protein by cutting with thrombin, factor-Xa, and such, as required.

The polypeptides of the invention may be produced by genetic engineering techniques, well-known peptide synthesizing methods, or, where the polypeptide is a wild type polypeptide that is mixed with or complexed with (*e.g.,* conjugated to) a polypeptide comprising a mutation, by excising the wild type protein from a natural source with a suitable peptidase. Recombinant techniques, synthesis methods, including solid-phase and liquid-phase synthesis methods, and techniques for isolating and digesting proteins are well known in the art and can be used in the context of the present invention.

Another objective of this invention is to provide DNA encoding a polypeptide described above. The DNA may be useful for producing the above proteins of the invention *in vivo* and *in vitro.* Furthermore, for example, it is also possible to use the DNA for application to gene therapy and such of diseases arising from abnormalities of the gene encoding the protein of the present invention. The DNA may be provided in any form as long as it encodes a protein of the invention. Thus, the DNA may be a cDNA synthesized from mRNA, genomic DNA, or chemically synthesized DNA. Furthermore, a DNA comprising any nucleotide sequence based on the degeneracy of genetic code may be included as long as it encodes a protein of the present invention.

*Nucleic Acids:* As used herein, an "isolated nucleic acid" is a nucleic acid, the structure of which is not identical to that of any naturally occurring nucleic acid or to that of any fragment of a naturally occurring genomic nucleic acid spanning more than three genes. The term therefore covers, for example, (a) a DNA which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, *i.e.,* a gene encoding a fusion protein. Specifically excluded from this definition are nucleic acids present in random, uncharaeterized mixtures of different RNA molecules, transfected cells, or cell-clones, *e.g.,* as these occur in DNA library such as a cDNA.or genomic DNA library.

Accordingly, in one aspect, the invention provides an isolated or purified nucleic acid molecule that encodes a polypeptide described herein. Preferably, the isolated nucleic acid molecule includes a nucleotide sequence that is at least 60% identical to the coding sequence shown in Fig. 1B (nucleotides 246-1622 of SEQ ID NO:26) or to the longer CD4 sequence of Fig. 1A (SEQ ID NO:26, which includes the coding sequence and flanking sequences). More preferably, the isolated nucleic acid molecule is at least 65%, 70%, 75%, 80%, 85%, 90%, 91%, 92%, 93%, 94%, 95%, 96%, 97%, 98%, 99%, or more, identical to the coding sequence shown in Fig. 1B or the longer CD4-associated nucleic acid of SEQ ID NO:26. Where the isolated nucleic acid molecule is shorter than the reference sequence, *e.g.*, shorter than SEQ ID NO:26 or the coding sequence thereof, the comparison is made to a segment of the reference sequence of the same length (excluding any loop required by the homology calculation) and following optimum alignment with the reference sequence.

As used herein, "% identity" of two amino acid sequences, or of two nucleic acid sequences, is determined using the algorithm of Karlin and Altschul (Proc. Natl. Acad. Sci. USA, 87:2264-2268, 1990), modified as in Karlin and Altschul (Proc. Natl. Acad. Sci. USA, 90:5873-5877, 1993). Such an algorithm is incorporated into the NBLAST and XBLAST programs of Altschul et al. (J Mol. Biol. 215:403-410, 1990). BLAST nucleotide searches are performed with the NBLAST program, score = 100, wordlength = 12. BLAST protein searches are performed with the XBLAST program, score = 50, wordlength = 3. To obtain gapped alignment for comparison purposes GappedBLAST is utilized as described in Altschul et al (Nucl. Acids Res. 25:3389-3402, 1997). When utilizing BLAST and GappedBLAST programs the default parameters of the respective programs (*e.g*., XBLAST and NBLAST) are used to obtain nucleotide sequences homologous to a nucleic acid molecule of the invention.

The DNA of the invention can be prepared by any method known to one skilled in the art. For instance, the DNA may be prepared by constructing a cDNA library from cells expressing the protein, and performing hybridization using a partial sequence of the DNA of the invention (SEQ ID NO:26, for instance) as a probe. A cDNA library may be constructed according to the method described in the literature (Sambrook et al., Molecular Cloning, Cold Spring Harbor Laboratory Press, 1989), or a commercial DNA library may be used. Alternatively, the DNA may be prepared by obtaining RNA from a cell expressing a protein of the present invention, synthesizing oligo DNA based on the sequence of the present DNA (SEQ ID NO:26, for instance), performing PCR using the synthesized DNA as the primer, and amplifying the cDNA encoding a protein of the present invention.

By determining the nucleotide sequence of the obtained cDNA, the translation region encoded by the cDNA can be determined, and the amino acid sequence of the protein of the present invention can be obtained. Furthermore, genomic DNA can be isolated by screening genomic DNA libraries using the obtained cDNA as a probe.

Specifically, this can be done as follows: First, mRNA is isolated from cells, tissues, and organs (for example, ovary, testis, placenta, etc.) expressing a protein of the invention. To isolate the mRNA, at first, whole RNA is prepared using well-known methods, for example, guanidine ultracentrifugation method (Chirgwin et al., Biochemistry, 18:5294-5299, 1979), the AGPC method (Chomczynski et al., Anal. Biochem., 162:156-159, 1987), and such, and mRNA from whole mRNA is purified using the mRNA Purification Kit (Pharmacia), etc. Alternatively, mRNA may be directly prepared using the QuickPr-ep mRNA Purification Kit (Pharmacia).

cDNA is synthesized using reverse transcriptase from the obtained mRNA. cDNA can be synthesized by using the AMV Reverse Transcriptase First-strand cDNA Synthesis Kit (SEIKAGAKU CORPORATION), etc. Additionally, cDNA synthesis and amplification may be also done by using the primer and such described herein following the 5'-RACE method (Frohman et al., Proc. Natl. Acad. Sci. USA, 85:8998-9002, 1988; Belyavsky et al., Nucl. Acids Res., 17:2919-2932, 1989) utilizing the polymerase chain reaction (PCR) and the 5'-Ampli FINDER RACE KIT (Clontech).

The objective DNA fragment is prepared from the obtained PCR product and ligated with the vector DNA. Thus, a recombination vector is created, introduced into *E. coli,* and such, and colonies are selected to prepare the desired recombination vector. The nucleotide sequence of the objective DNA can be verified by known methods, for example, the dideoxy nucleotide chain termination method.

Regarding the DNA of the invention, a sequence with higher expression efficiency can be designed by considering the codon usage frequency in the host used for the expression (Grantham et al., Nucl. Acids Res., 9:43-74, 1981). The DNA of the invention may also be modified using commercially available kit and known methods. Modifications are given as, for example, digestion by restriction enzymes, insertion of synthetic oligonucleotides and suitable DNA fragments, addition of linkers, insertion of a start codon (ATG) and/or stop codon (TAA, TGA, or TAG), and such.

Stringent conditions can be suitably selected by one skilled in the art, and for example, low-stringent conditions can be given. Low-stringent conditions are, for example, 42°C, 2x SSC, and 0.1% SDS, and preferably 50°C, 2x SSC, and 0.1% SDS. More preferable are highly stringent conditions which are, for example, 65°C, 2x SSC, and 0.1% SDS. Under these conditions, the higher the temperature, the higher the homology of the obtained DNA will be. The above DNA hybridizing to the DNA with the sequences of SEQ ID NO:26, is preferably a natural DNA such as cDNA and chromosomal DNA.

Moreover, the present invention provides a vector containing a DNA of the invention as an insert. The vector may be useful for maintaining the DNA in host cells or producing the protein of the invention.

If the host cell is *E. coli* (such as JM109, DH5α, HB101, and XL1Blue), any vector may be used as long as it contains the "ori" for amplification in *E. coli* that enables large-scale preparation, and a selection marker for transformants (for instance, a drug resistance gene that enables selection by a drug such as ampicillin, tetracycline, kanamycin, and chloramphenicol). For instance, series of the M13 vectors and pUC vectors, pBR322, pBluescript, pCR-Script, and so on may be used. For the purpose of subcloning or excision of a cDNA, pGEM-T, pDIRECT, pT7, and such may be used as well. For producing the protein of the invention, an expression vector is especially useful. For instance, if the protein is to be expressed in *E. coli,* the expression vector must have such characteristics as above to be amplified in *E. coli,* and a promoter for efficient expression, such as the lacZ promoter (Ward et al., Nature 341:544-546, 1989; FASEB J., 6:2422-2427, 1992), araB promoter (Better et al., Science 240:1041-1043, 1988), or T7 promoter. Such vector includes pGEX-5X-1 (Pharmacia), vectors in the QIAexpress system (QIAGEN), pEGFP, pET (BL21 expressing the T7 RNA polymerase is favorably used as the host), and so on except those mentioned above.

The vector may contain a signal sequence for polypeptide secretion. The pelB signal sequence (Lei et al., J. Bacteriol., 169:4379, 1987) may be used to produce the proteins in the periplasm of *E. coli.* Vectors may be introduced into host cells, for example, by the calcium chloride method or electroporation.

For example, the expression vector to prepare the protein of the invention may be a mammal-derived expression vector *(e.g.,* pcDNA3 (Invitrogen), pEGF-BOS (Nucl. Acids. Res. 18:5322, 1990), pEF and pCDM8), an insect cell-derived expression vector (e.g., "Bac-to-BAC baculovirus expression system"(GIBCO BRL), pBacPAK8), a plant-derived expression vector (*e.g.,* pMH1 and pMH2), an animal virus-derived expression vector (*e.g.,* pHSV, pMV, and pAdexLcw), a retrovirus-derived expression vector (*e.g.,* pZIpneo), an yeast-derived expression vector (*e.g.,* "Pichia Expression Kit " (*In vitrogen*), pNV11 and SP-Q01), or a Bacillus subtilis-derived expression vector (*e.g.,* pPL608 and pKTH50), other than *E. coli.*

For the expression in animal cells, such as CHO, COS, and NIH3T3 cells, the expression vector must have a promoter such as SV40 promoter (Mulligan et al., Nature, 277:108, 1979), MMLV-LTR promoter, EF1α promoter (Mizushima et al., Nucl. Acids Res., 18:5322, 1990), and CMV promoter. More favorably, the vector may contain a marker for the selection of transfected cells (for instance, a drug resistance gene for selection by a drug such as neomycin and G418). Such vectors include pMAM, pDR2, pBK-RSV, pBK-CMV, pOPRSV, pOP13, and so on.

Furthermore, in order to achieve stable gene expression and amplification of the copy number of genes in cell, CHO cells deficient in the metabolic pathway for nucleotide synthesis may be used. The CHO cell is transfected with an expression vector containing the DHFR gene that complements the deficiency (such as pCHOI), then the vector may be amplified by methotrexate (MTX) treatment. For transient gene expression, COS cells containing a gene expressing the SV40 T-antigen on its chromosome may be used to transform with a vector containing the SV40 replication origin (such as pCD). Examples of replication origins to be used in the present invention include those derived from polyomavirus, adenovirus, bovine papillomavirus (BPV), and such. Moreover, to amplify the gene copies in host cell lines, the expression vector may include an aminoglycoside transferase (APH) gene, thymidine kinase (TK) gene, *E. coli* xanthine guanine phosphoribosyl transferase (Ecogpt) gene, dihydrofolate reductase (dhfr) gene, and such as a selective marker.

*In vivo* expression of the DNA of the invention may be performed by constructing the DNA into an appropriate vector and transfecting the construct into the body using retrovirus, liposome, cationic liposome, adenovirus, and so on. It is possible to use such a construct to perform gene therapy for diseases that arise from mutations in the NR10 gene. Examples of vectors used for this purpose include an adenovirus vector (such as pAdexlcw) and a retrovirus vector (such as pZIPneo), but are not limited thereto. General manipulations, such as insertion of the DNA into the vector, may be performed by using standard methods (Molecular Cloning, 5.61-5.63). The vector may be administered to the patient through *ex vivo* or *in vivo* methods.

*Production of Selenocysteine-containing proteins:* Selenocysteine is incorporated into proteins co-translationally, in all systems studied to date, by "re-coding" of the canonical UGA stop-codon. This process is mediated by several cis- and trans-acting elements that converge on the ribosomal-mRNA complex during translation, effecting selenocysteine insertion at UGA codons instead of polypeptide chain termination. Sec-insertion has been well-described in *E. coli,* and occurs (in this organism) when a particular secondary RNA stem-loop structure, termed the Sec-Insertion Sequence (SECIS), is positioned at a defined position on the 3' side of a UGA codon located within mRNA. The detailed process of Sec-insertion in *E. coli* has been described in a seminal paper by Arner and colleagues (Arner et al., J. Mol. Biol. 292:1003-1016, 1999), and this work provided the basis for generic production of any theoretical selenocysteine-containing protein for which a SECIS-containing encoding mRNA can be designed.

While recombinant expression methods have since been developed to enable reasonably efficient selenoprotein synthesis in *E. coli,* a singular problem of this method is that the incorporation of the SECIS element into the reading frames of genes results in a sequence change involving 4-5 amino acids on the C-terminal side of the inserted Sec residue. Thus, this method may be limiting in cases where a single point substitution with U is desirable, and/or the amino acid changes have significantly deleterious effects on protein structure/function. Nevertheless, functional Sec-containing recombinant proteins have been expressed and purified in *E. coli* using this method (Jiang et al., Biochem. Biophys. Res. Comm. 321:94-101, 2004).

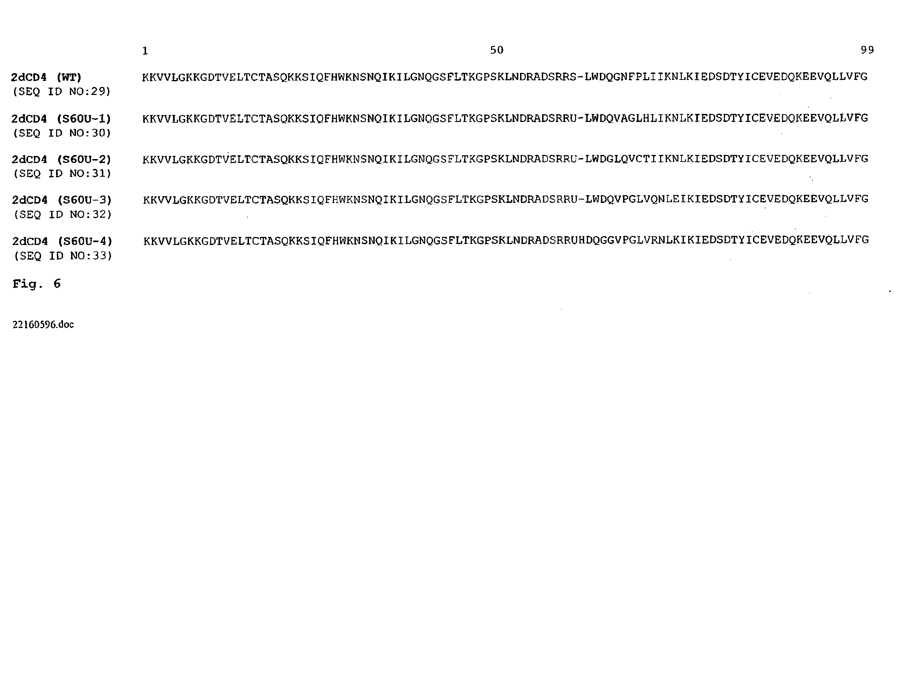

We applied these methods to the production of recombinant CD4 molecules containing S60U mutations. We generated a panel of protein expression vectors encoding 4 different 2dCD4 (S60U) proteins (and 4 corresponding control vectors). The encoded 2dCD4 sequences are shown in Fig. 6, and show the unique amino acid changes incurred in these four proteins as a result of the introduction of SECIS elements into the 2dCD4 ORFs downstream of the Sec60-encoding stop-codon. The particular sequences of the four Seleno-CD4-encoding genes were designed attempting to limit, as much as possible according to the constraints defined by the SECIS element, the amino acid changes to biochemically conservative ones. Thus the production of alternative Sec-CD4 proteins based on manipulating, where feasible, the position and sequence of the SECIS element in the CD4 ORF, is possible.

The process of Sec-incorporation in mammalian expression systems is distinguishable from that which occurs in *E. coli* to a large extent by the required position of the SECIS element. In mammalian cells, the SECIS element usually occurs outside the reading frame of the gene into which Sec is inserted, at positions that can be several thousand bases downstream of the re-codable UGA, and well outside the mRNA coding region. Thus, the advantage of such a system is that it would enable the generation of a recombinant protein containing a single Sec substitution, with no other amino acid changes incurred through a SECIS engineered into the coding region. While this basic difference between selenoprotein expression in mammalian cells and *E. coli* is well-defined, the methodology for producing recombinant selenoproteins in mammalian expression systems is not firmly established, and the efficiency of protein expression in these hosts generally several orders of magnitude lower than in *E. coli.* Nevertheless, a recent study reported the production of milligram quantities of a recombinant selenoprotein from a mammalian cell expression system (Hofer et al., Proc. Natl. Acad. Sci. USA 105:12451-12456, 2008).

The invention features mutant CD4 polypeptides that include a selenocysteine residue at a position corresponding to position 85 of SEQ ID NO:22 (position 60 in the mature form of CD4; position 3 in any of SEQ ID NOs.:2, 3, 6, or 7-9) and nucleic acids that encode them. The polypeptides can be generated using a SECIS element as described above and known in the art. Representative polypeptides are shown in Fig. 6. More generally, the invention features mutant CD4 polypeptides that include one or more mutations on the C-terminal side of the serine residue at position 85 of SEQ ID NO:22. For example, the polypeptide can include one or more contiguous residues from residues 1-84 of SEQ ID NO:22 (*e.g.,* one or more of residues 26-84 of SEQ ID NO:22) followed by a selenocysteine residue, followed by one or more mutations in the region corresponding to residues 86-99 of SEQ ID NO:22, followed by one or more residues of the wild type CD4 from residue 100 to the C-terminus of SEQ ID NO:22. The length of the wild type sequence flanking the selenocysteine residue and the variable region constituting residues 86-99 can vary. For example, the N-terminus of the polypeptide can include 0, 1-5, 6-10, 11-15, 16-20, 21-25, 26-30, 31-35, 36-40, 41-45, or 46-50 residues of the wild type CD4 sequence. Similarly, the C-terminus of the polypeptide can include 1, 1-5, 6-10, 11-15, 16-20, or more residues, including all of the residues, that constitute the wild type N-terminal sequence following residue 99 of SEQ ID NO:22. The variable region following the selenocysteine can vary greatly from the wild type sequence. More specifically, the variable region can be at least 10%, 20%, 30%, 40%, 50%, 60%, 70%, 80%, or 90% different from the corresponding wild type sequence. Polypeptides in which the sequence C-terminal to the selenocysteine varies from wild type as described here include those that specifically bind gp120.

Another object of this invention is to provide a transformant that contains the DNA of the invention in an expressible manner. The host cell to insert the vector of the present invention is not limited in any way, and *E. coli,* a variety of animal cells, and so on may be used. The transformant may be used as a producing system for preparing or expressing a protein of the invention. *In vitro* and *in vivo* production systems are known as production systems for producing proteins. Production systems using eukaryotic cells and prokaryotic cells may be used as the *in vitro* production systems.

When using eukaryotic cells, production systems using, for example, animal cells, plant cells, and fungal cells are available as hosts. Examples of animal cells used include mammalian cells such as CHO (J. Exp. Med., 108:945, 1995), COS, 3T3, myeloma, baby hamster kidney (BHK), HeLa, Vero, amphibian cells such as Xenopus oocytes (Valle et al., Nature, 291:338-340, 1981), insect cells such as sf9, sf21, or Tn5. As CHO cells, especially DHFR gene-deficient CHO cell, dhfr-CHO (Proc. Natl. Acad. Sci. USA, 77:4216-4220, 1980), and CHO K-1 (Proc. Natl. Acad. Sci. USA, 60:1275, 1968) can be suitably used. For large-scale preparation in animal cells, CHO cells may be favorably used. The vector may be transfected into host cells using a variety of methods, such as those using calcium phosphate, DEAE-dextran, or cationic liposome DOTAP (Boeringer Mannheim), as well as electroporation, lipofection, and so on.

Nicotiana tabacum-derived cells are well known as protein production systems in plant cells, and these can be callus cultured. As fungal cells, yeasts such as the Saccharomyces genus, for example, Saccharomyces cerevisiae; filamentous bacteria, such as Aspergillus genus, for example, Aspergillus niger are known.

Bacterial cells may be used as prokaryotic production systems. As bacterial cells, *E. coli,* for example, JM109, DH5α, HB101, and such, as well as others like *Bacillus subtilis* are known.

Proteins can be obtained by transforming these cells with the objective DNA, and culturing the transformed cells *in vitro* according to known methods. For example, DMEM, MEM, RPMI1640, and IMDM can be used as culture media of animal cells. Occasionally, fetal calf serum (FCS) and such serum supplements may be added in the above media; alternatively, a serum-free culture medium may be used. The pH is preferably from about 6 to 8. The culturing is usually performed at about 30°C to 40°C, for about 15 to 200 hr, with medium changes, aeration, and stirring taking place as necessary.

On the other hand, and for example, production systems using animals and plants may be given as *in vivo* protein production systems. The objective DNA is introduced into the plant or animal, and the protein is produced within the plant or animal and then recovered. We may use the term "host" when referring to such animals and plants.

When using an animal host, mammalian and insect production systems can be used. Mammals such as goats, pigs, sheep, mice, and cows may be used (Vicki Glaser, SPECTRUM Biotechnology Applications (1993)). Transgenic animals may also be used to express the present polypeptides.

For example, the objective DNA is prepared as a fusion gene with a gene encoding a protein intrinsically produced into milk, such as goat β casein. Next, the DNA fragment containing the fusion gene is injected into goat's embryo, and this embryo is implanted in female goat. The objective protein can be collected from the milk of the transgenic goats produced from the goat that received the embryo, and descendants thereof. To increase the amount of protein-containing milk produced from the transgenic goat, a suitable hormone/hormones may be given to the transgenic goats (Ebert et al., Bio/Technology, 12:699-702, 1994).

Silk worms may be used as insects. When using silk worms, they are infected with baculoviruses to which the DNA encoding objective protein has been inserted, and the desired protein can be obtained from the body fluids of the silk worm (Susumu et al., Nature, 315:592-594, 1985).

When using plants, for example, tobacco can be used. In the case of tobacco, the DNA encoding the objective protein is inserted into a plant expression vector, for example, pMON 530, and this vector is introduced into a bacterium such as Agrobacterium tumefaciens. This bacterium is infected to tobacco, for example, Nicotiana tabacum, and it is able to obtain the desired polypeptide from the tobacco leaves (Julian et al., Eur: J. Immunol. 24:131-138, 1994).

Thus-obtained protein of the invention is isolated from inside or outside (medium, etc.) the host cell, and may be purified as a substantially pure homogenous protein. The separation and purification of the protein can be done using conventional separation and purification methods used to purify proteins and are not limited to any specific method. For example, chromatography column, filtration, ultrafiltration, salting out, solvent precipitation, solvent extraction, distillation, immunoprecipitation, SDS-polyacrylamide gel electrophoresis, isoelectric focusing, dialysis, recrystalization, and such may be suitably selected, or combined to separate/purify the protein.

For example, affinity chromatography, ion exchange chromatography, hydrophobic chromatography, gel filtration, reversed-phase chromatography, adsorption chromatography, and such can be exemplified as chromatographies (Strategies for Protein Purification and Characterization: A Laboratory Course Manual. Ed Daniel R. Marshak et al., Cold Spring Harbor Laboratory Press, 1996). These chromatographies can be performed by liquid chromatography, such as HPLC, FPLC, and the like. The present invention encompasses proteins highly purified by using such purification methods.

Proteins can be arbitrarily modified, or peptides may be partially excised by treating the proteins with appropriate modification enzymes prior to or after the purification. Trypsin, chymotrypsin, lysyl endopeptidase, protein kinase, glucosidase, and such are used as protein modification enzymes.

*gp120-CD4 complex:* In certain aspects, the gp120 and CD4 complexes are hybrid proteins including any of the gp120 and CD4 polypeptide sequences described above. For example, the CD4 sequence may precede or follow the gp120 sequence. The gp120 sequence may be associated with or part of other Env sequences (*e.g.,* gp40). Further, any of the hybrid proteins may include one or more linkers of varying length *(e.g.,* 3 to 30 residues in length, or any integer number of residues between 3 and 30). In some embodiments, the gp120-CD4 hybrid proteins are encoded by a single open reading frame in a nucleic acid sequence.

Methods for producing gp120-CD4 hybrid proteins are described in International Publication No. 2004/037847.

The gp120 and CD4 complexes may be produced as follows. As described, gp120 and CD4 polypeptides can be produced as hybrid proteins and further linked to each other by the formation of one or more specific covalent bonds. In some embodiments, gp120 and CD4 proteins can be separately produced and complexed to each other by the formation of one or more specific covalent bonds (*e*.*g*., disulfide, trisulphide, diselenide, or selenosuphide bonds) or by non-covalent forces.

In addition, suitable complexes may be produced by, for example, co-transfecting host cells with constructs encoring hybrid gp120-CD4 proteins, gp120, and CD4 polypeptides of the present invention. Co-transfection can be accomplished either in trans or cis, *i.e.,* by using separate vectors or by using a single vector that bears both of the gp120 and CD4 polypeptide (or fragment). If done using a single vector, both genes can be driven by a single set of control elements. A single set of control elements is preferably employed in the case of constructs encoding gp120-CD4 hybrid proteins (see for example, Example 4). Alternatively, the gp120 and CD4 encoding sequences can be present on the vector in individual expression cassettes, driven by individual control elements. Following expression, the polypeptides may spontaneously associate. Alternatively, the complexes can be formed by obtaining polypeptides (which have been produced separately) and mixing them together. The polypeptides may be substantially purified or not purified at all. International Publication No. WO 96/04301, published February 15, 1996, describes such complexes.

The formation of covalent bonds between gp120 and CD4 polypeptides (or polypeptide sequences) may be performed under conditions that are conducive to formation of such bonds (for example, oxidizing conditions for the formation of disulfide bonds). Standard aqueous solutions and conditions conducive for oxidation of cysteine are known in the art.

*Antibodies:* The present invention also provides antibodies that specifically bind to a polypeptide of the invention and/or to a complex containing such polypeptides. The antibodies may be those elicited in response to gp120 when a CD4 polypeptide is complexed with gp120 and used as an immunogen (be they neotope based antibodies or simply antibodies generated to sites exposed on gp120 by the polypeptide binding). These antibodies could be useful as therapies, microbicides or as diagnostic reagents.

*Pharmaceutical Compositions:* The polypeptides described herein, including those in an "extended" form *(e.g.,* a core polypeptide within a fusion protein or a covalently bound complex with a heterologous polypeptide (*e.g.,* gp120)), can be formulated as pharmaceutical compositions. In certain embodiments, the pharmaceutical compositions can be used to elicit or enhance an immune response against an HIV antigen (*e*.*g*., gp120), while in others, the polypeptides (*e.g.,* a core polypeptide) can be used to inhibit binding of an HIV virion to a T cell, thereby inhibiting infection of that cell.

Our expectation is that the present compositions will be formulated for intravenous, nasal, or sublingual administration, but the invention is not so limited. For example, according to the need, the drugs can be taken orally, with protective coatings to inhibit degradation in the stomach.

When formulated for injection (*e.g.,* intravenous injection) the polypeptides can be formulated in a sterile solution or suspension with water or any other pharmaceutically acceptable liquid. For example, the compounds can be mixed with pharmacologically acceptable carriers or media (*e.g.,* sterilized water, physiological saline, plant-oil, emulsifiers, solvents, surfactants, stabilizers, flavoring agents, excipients, vehicles, preservatives and binders, optionally in a unit dose form required for generally accepted drug implementation).

Examples of additives that can be mixed to form tablets, capsules, and the like, are: binders such as gelatin, corn starch, tragacanth gum and gum arabic; excipients such as crystalline cellulose; swelling agents such as corn starch, gelatin and alginic acid; lubricants such as magnesium stearate; sweeteners such as sucrose, lactose or saccharin; and flavoring agents such as peppermint, Gaultheria adenothrix oil and cherry. When the unit dosage form is a capsule, a liquid carrier, such as oil, can also be included.

Sterile composites for injections can be formulated following normal drug implementations using vehicles such as distilled water used for injections. For example, physiological saline, glucose, and other isotonic liquids including adjuvants, such as D-sorbitol, D-mannnose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as Polysorbate 80 (TM) and HCO-50.

Methods well known to one skilled in the art may be used to administer the pharmaceutical compound to patients, for example as intraarterial, intravenous, percutaneous injections and also as intranasal, transbronchial, intramuscular or oral administrations. The dosage and method for administration vary according to the body-weight and age of the patient, the administration method, and such, but one skilled in the art can suitably select these parameters. Dosages (*e.g.,* injected doses) to an adult (body weight, 60 kg) can range from 100 µg to 10-20 mg per day.

A "therapeutically effective amount" of the compositions described here is an amount sufficient to prevent, reduce the risk of, or provide relief from HIV infection, AIDS, and/or the symptoms of HIV infection and AIDS (which are known in the art).

When the protein is administered parenterally in the form of an injection to a standard adult (weight 60 kg), although there are some differences according to the patient, target organ, symptoms and method of administration, it is convenient to intravenously inject a dose of about 0.01 mg to about 30 mg per day, preferably about 0.1 to about 20 mg per day, and more preferably about 0.1 to about 10 mg per day.

The compositions described herein may be administered with other commercially available anti-retroviral drugs used in the treatment of HIV infection (for example, reverse transcriptase inhibitors, nucleotide analogs, protease inhibitors, integrase inhibitors, and/or fusion inhibitors). The compositions may also be administered in conjunction with combination therapies such as highly active anti-retroviral therapy (HART).

Particularly when used to elicit or enhance an immune response, the present compositions can be administered in conjunction with other antigens and/or immunoregulatory agents (for example, immunoglobulins, cytokines, lymphokines, and chemokines, including but not limited to IL-2, modified IL-2 (Cys125 to Ser125 substitution mutant), GM-CSF, IL-12, alpha- or gamma-interferon, IP-10, MIP1 and RANTES).

The compositions may be administered as polypeptides or as naked nucleic acid vaccines using viral vectors (*e.g.,* retroviral vectors, adenoviral vectors, adeno-associated viral vectors, alphaviral vectors) or non-viral vectors (*e.g.,* liposomes, particles coated with nucleic acid or protein). The compositions may comprise a mixture of proteins and nucleic acids.

A carrier that does not itself induce the production of antibodies harmful to the individual receiving the composition is optionally present. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. These carriers are well known to one of ordinary skill in the art. The Env polypeptide and/or gp120 polypeptide may be conjugated to a bacterial toxoid (for example, toxoid from diphtheria, tetanus, or cholera).

Adjuvants may be used to enhance effectiveness. Non-limiting examples of adjuvants include: (1) aluminum salts (for example, aluminum hydroxide, aluminum phosphate, and/or aluminum sulfate); (2) oil-in-water emulsion formulations (for example, MF59 as described in International Publication No. WO 90/14837); (3) saponin adjuvants; (4) Complete Freunds Adjuvant (CFA) and Incomplete Freunds Adjuvant (IFA); (5) cytokines (for example, IL-1 and/or IL-2), and (6) non-toxic mutants of toxin proteins (for example, cholera toxin, pertussis toxin, and/or *E. coli* heat labile toxin).

Muramyl peptides as described in International Publication No. WO 04/037847 are also used as adjuvants.

Microparticles as described in International Publication No. WO 04/037847 can be used as adjuvants and/or delivery vehicles.

Typically, vaccine compositions are prepared as injectables (either liquid solutions or suspension). Solid form suitable for solvation or suspension in liquid prior to administration to an individual are part of this invention.

The vaccines of this invention will comprise a therapeutically effective amount (as will the compositions of this invention). The phrase "therapeutically effective amount" can mean that the polypeptides or complexes described herein are delivered in an amount sufficient to induce a protective immune response in an uninfected, infected, or unexposed individual to whom it is administered. Such an immune response will generally result in the development in the subject of a secretory, cellular, and/or antibody-mediated immune response to the vaccine. Usually, such a response includes but is not limited to one or more of the following: (1) antibodies of isotypes A, D, E, G, or M are produced; (2) the proliferation of B and T lymphocytes; (3) the induction of activating, growth, and differentiation signals to immune cells, and (4) the expansion of helper, suppressor, and/or cytotoxic T cell populations.

The therapeutically effective amount will vary depending on the subject being treated. Variables between individuals to be treated include but are not limited to the age and general condition of the individual, the capacity of the individual's immune system to synthesize antibodies, the degree of protection desired, the severity of the condition being treated, and the mode of administrations. A "therapeutically effective amount" will be understood to be the amount that confers a therapeutic benefit on the recipient, and the precise amount(s) can be determined through routine trials.

### EXAMPLE 1

### General Methods.

Peptides, MAbs and recombinant proteins. Peptides (N-terminal amine, C-terminal amide) were synthesized using Fmoc-based solid phase synthesis methods, purified by HPLC to purities of greater than 90%, and analysed by mass spectrometry (JPT Peptide Technologies, Berlin, Germany). The following reagents were obtained through the NIH AIDS Research and Reference Program, Division of AIDS, NIAID, NIH (contributors in parentheses): MAb F425 B4a1 (Drs. M. Posner and L. Cavacini); MAb 2G12 (Dr. H. Katinger); MAb IgG1 b12 (Drs. D. Burton and C. Barbas); MAb 654-30D (Dr. S. Zolla-Pazner); sCD4 (Dr. N. Schuelke); sCD4-183 (2dCD4) (Pharmacia); and recombinant HIV-1BaL gp120 (Dr M. Reitz).

Peptide analyses. Synthetic 7-mer peptides Hel-S (RRSLWDQ, SEQ ID NO:10), Hel-Cs (DLRQCWR, SEQ ID NO:27) and Hel-C (RRCLWDQ, SEQ ID NO:2) were analysed by discontinuous PAGE according to the method of Schagger and von Zagau with modifications. Briefly, 2.5 µg of each lyophilized peptide reconstituted in pure H₂O was mixed with a reducing agent-free loading buffer (0.1 M Tris, 20% glycerol, 8% SDS and 0.02% Coomassie blue G-250), incubated at 80°C for 3 minutes and loaded onto a 17.5% Tris/Tricine gel. Samples were resolved at 20 mAper gel until stacked (1-2 hours) and at 35 mA for 16 hours through the resolving gel. The gel was fixed by incubation in a 5% glutaraldehyde solution for 1 hour and then stained with Coomassie Blue-R250. Peptide monomer/dimer ratios were calculated by densitometry using Quantity One® software (BioRad, Hercules, CA (USA)). To evaluate the redox states of cysteine-containing peptides further, reduced Glutathione (GSH) (Sigma, UK), β-mercaptoethanol and dithiothreitol (DTT) were included in the sample loading buffer at concentrations ranging from 0 to 400 mM, and the samples were resolved and analysed as described above.

Peptide N-terminal [¹⁴C]-Acetylation. Hel-S, Hel-Cs and Hel-C were radioactively labeled by N-terminal acetylation according to the method of Fraenkel-Conrat and Colloms with modifications. Briefly, peptides were reconstituted in an ice cold saturated solution of sodium acetate (> 3 M) to a concentration of 5 mM. [¹⁴C]-Acetic anhydride (specific activity 116 mCi/mmol, Amersham Biosciences, UK) was then added to 100 µl of the reconstituted peptide solution in two 1 µl installments over 15 minutes, and the labeling reactions were incubated for 2 hours on ice. After addition of the final acetic anhydride installment, the reaction concentrations of both peptide and acetic anhydride were 5 mM, and the samples contained 1% toluene and 500 nCi/µl of [¹⁴C]. An equal volume (100 µl) of ice cold PBS (pH 7.4) was then added to each reaction. For buffer exchange and removal of unincorporated radiolabel, the [¹⁴C]-labeled peptides were purified chromatographically using Sephadex G-10 spin columns (Sigma, UK) equilibrated in PBS (pH 7.4). The concentration of the purified labeled peptides was determined by measuring A280, and the labeling efficiencies were analysed by discontinuous PAGE and fluorescence autoradiography, as well as by liquid scintillation counting using Packard Tri-Carb 2200 Liquid Scintillation Analyzer. Labeled peptides were stored in 50 µl aliquots at -80°C.

Electrophoretic Mobility Shift Assay (EMSA). Peptide-gp120 binding reactions were set up in 50 µl and contained 5 µM of pure recombinant gp120BaL and 500 µM of radio labeled Hel-S, Hel-Cs or Hel-C in PBS (pH 7.4). Reactions were incubated at 25°C for 3 hours and then stopped by the addition of an equal volume (50 µl) of either reducing agent-free loading buffer (0.1 M Tris, 20% glycerol, 8% SDS and 0.02% Coomassie blue G-250) or the same buffer containing 1, 10 or 100 mM of DTT. The samples were incubated at 80°C for 3 minutes before loading on a 20 × 20 cm 6 - 20% gradient Tris/Tricine polyacrylamide gel, and were resolved at a constant 40 V for 16 hours at room temperature. After electrophoresis, the gel was rinsed thoroughly with H₂O and then stained lightly by incubation in a solution containing 10% methanol, 10% acetic acid and 0.02% Coomassie Blue R-250 for 10 minutes with gentle shaking. The gel was rinsed with H₂0, and bands corresponding to gp120 were excised using a gel punch and placed in 1.5 ml microcentrifuge tubes. The gp120-containing polyacrylamide slices were solubilized by incubation at 37°C for 16 hours in 400 µl of a solution containing 30% H₂O₂ and 0.03% ammonium hydroxide. The dissolved polyacrylamide samples were added to 2 ml of Aquasol-2 scintillation cocktail (Perkin Elmer, USA), and radioactive emissions expressed as counts per minute (cpm) measured by scintillation counting.

Surface Plasmon Resonance. All surface Plasmon resonance (SPR) experiments were performed on a BIA3000 optical biosensor (Biacore Inc., Uppsala, Sweden). Recombinant gp120BaL was immobilized on CM5 sensor chips by amine coupling according to the manufacturer's instructions. Briefly, a solution of 0.2 M EDC and 0.05 M NHS was used to activate carboxyl groups on the sensor surface at a flow rate of 5 µL/min for 10 minutes. Recombinant gp120BaL, dissolved in 100 mM sodium acetate buffer (pH 5.5) to a final concentration of 0.05 µM, was passed over the sensor chip surface at a flow rate of 5 µL/min until an immobilization level of 5000 RU was achieved. Excess carboxyl groups were capped by injection of 1 M ethanolamine (pH 8.0) at a flow rate of 5 µL/min for 5 minutes. Unbound ligand was removed by injecting 10 mM glycine (pH 2.5) at a flow rate of 5 µL/min for 2 minutes. In all experiments, the neighbouring flow cell was treated as mentioned above without the immobilization of gp120BaL, and this served as a reference surface. Prior to all experiments, the functionalities of the gp120 surfaces were tested by binding sCD4 at 500 nM in HEPES-buffered saline supplemented with 0.15 M NaCl (HBS-N, pH7.4). Analyte solutions were prepared in HBS-N at least 30 minutes prior to injection. All binding experiments were performed in duplicate at 25°C. For peptide binding analyses, increasing concentrations of peptide (from 0 to 500 µM) were passed over the surface at a flow rate of 5 µL/min, with a 5 minute association phase and a 6 minute dissociation phase. Surfaces were regenerated with a 20 mM sodium hydroxide (NaOH) solution between consecutive peptide analyses. The integrity of the surfaces was tested after each regeneration for sCD4 binding. Buffer injections and control surface binding were subtracted for all reported data.

Epitope Occlusion Assay. 96-well Maxisorp microtiter plates (Nunc, Denmark) were coated with gp120BaL (100 ng/mL in PBS, pH 7.4) for 3 hours at room temperature (100 µL per well). The coating solution was removed and plates were then blocked with 250 µL of PBS containing 0.05% Tween-20 and 10 mg/mL PBS (PBS-TB) for 16 hours at 4°C. After removing the blocking buffer and rinsing once with PBS containing 0.05% Tween-20 (PBS-T), 2dCD4 (0 to 1 µM) or peptides (0 to 1 mM) in PBS-T were added to the wells (50 µl in PBS-T), and plates were incubated at 25°C for 30 minutes. When checking the effect of reducing agents on the ability of the peptides to compete with antibody binding, the peptides were pre-formulated in PBS-T containing 10 mM of β-mercaptoethanol or DTT. MAbs F425 B4a1, 2G12, IgG1 b12 or 654-30D were then added to the wells as indicated (50 µl, final concentration 0.2 nM in PBS-T), and plates were incubated for a further 3 hours at 25°C. Plates were washed 5 times with 250 µL of ice-cold PBS-T, and 100 µL of an HRP-conjugated monoclonal anti-human Fc antibody (Amersham Biosciences, UK) diluted 1:2000 in PBS-T was then added to each well. Following incubation at 4°C for 1 hour, the plates were washed 5 times with ice-cold PBS-T. Bound secondary antibodies were detected by chromogenic methods using TMB Ultra substrate (PIERCE, IL USA) and quantified spectrophotometrically by measuring A450 levels. All microtiter plate manipulations (coating, washing, sample and reagent addition) were performed using an epMotion 5070 Liquid Handling System (Eppendorf, Hamburg Germany).

### EXAMPLE 2

Peptide Analyses. We analysed the oxidation states of the synthetic peptides Hel-S, Hel-Cs and Hel-C following reconstitution in PBS (pH 7.4) by high resolution Tris/Tricine PAGE (resolving buffer pH = 8.0). As expected, in aqueous solution at close to neutral pH, the wild-type CD4-mimetic peptide Hel-S consisted of a pure monomer of molecular weight equal to approximately 1 kDa (predicted Mw = 976 g/mol). In contrast, Hel-C, which contains a cysteine residue at position 3 (position 85 in SEQ ID NO:23; position 60 when the signal sequence (amino acid residues 1-25) is absent), and the scrambled peptide Hel-Cs, which contains a cysteine at position 5, consisted of a mixture of monomers and dimers. The latter are evident as bands of approximately 2 kDa, which presumably result from intermolecular disulfide bond formation. Densitometric analysis indicated that under these conditions, less than 10% of the Hel-Cs and Hel-C peptides existed as monomers. We then treated Hel-C with the thiol-containing reducing compounds β-mercaptoethanol (β-ME) and 1,4-dithiothreitol (DTT) to determine relative effects on cysteine oxidation in this peptide. DTT significantly reduced disulfide bond formation, increasing the proportion of Hel-C monomer to 50% at 10 mM, and completely abolished the ability of the peptide to oxidize at 200 mM. The ability of β-ME to prevent oxidation of the thiol groups on Hel-C was less marked, with β-ME requiring a concentration of between 25 and 50 mM to achieve a 1:1 monomer:dimer ratio, and unable to monomerize the peptide completely even at 200 mM. These data are consistent with the respective reduction potentials of DTT and β-ME, with DTT (-0.33 mV) an approximately twofold stronger reductant than β-ME (-0.17 mV) under standard conditions.

### EXAMPLE 3

Hel-C Binds gp120 through disulfide linkage on gp120. We adopted two independent approaches to evaluate the potential of Hel-C to bind recombinant gp120. First, we used an electrophoretic mobility shift assay (EMSA) to detect gp120-bound peptides following resolution of peptide/protein mixtures on denaturing gradient polyacrylamide gels. This method also enabled us to determine whether the mechanism of binding could be related to the establishment of disulphide bonds between the peptides and target gp120, by measuring the proportion of bound peptide in response to increasing doses of DTT. For detection, we decided to incorporate as small a label as possible into the peptide, so that steric effects of the labeling procedure would be minimal (such as would be the case with larger fluorescent/antigenic tags), and that the peptide would be able to adopt a secondary structure as close as possible to the R59-Q64 α-helix chain in CD4. We therefore labeled Hel-C and the control peptides Hel-S and Hel-Cs with [¹⁴C]-acetic anhydride, which incorporated a single [¹⁴C]-acetyl group on the peptide N-termini. The detection limit of the labeled peptides by autoradiographic methods was approximately 1 µmol, and stoichiometric constraints posed on detecting gp120-bound peptides in this manner prompted us to use liquid scintillation counting as an alternative more sensitive method of detecting radioactivity in resolved protein-peptide mixtures. Following resolution of the peptide-gp120 complexes on a 6-20% gradient SDS-Tris/Tricine gel, we were able to detect a significant quantity of Hel-C associated with excised gp120, while there was no detectable Hel-S bound to gp120. We also detected a small amount of the control scrambled peptide Hel-Cs associated with gp120, suggesting that a fraction of the cysteine-containing peptides may bind by reacting non-specifically with accessible thiols on target gp120. In order to gain insight into the nature of the association between gp120 and Hel-C, we treated the complexes with increasing doses of DTT, and found that the amount of Hel-C bound to gp120 decreased with increasing amounts of DTT added. Notably, the amount of bound peptide in the presence of 100 mM DTT was only 10% of the amount bound in the absence of any reducing compound, consistent with our findings that the cysteine of Hel-C is almost completely unable to oxidize in solutions containing 100 mM DTT.

We then sought to confirm these data by SPR analysis using a Biacore 3000 instrument. Recombinant gp120BaL was immobilized on CM5 sensorchips and exposed to increasing concentrations of Hel-S, Hel-Cs or Hel-C. We first used the 12-mer peptide 12p1 (RINNIPWSEAMM (SEQ ID NO:28), which was previously shown by SPR technology to bind gp120, as a quality control for our binding assays (Ferrer and Harrison, J. Virol. 73: 5795-5802, 1999).

At just over 100 µM, the amount of 12p1 bound to a 5000 RU gp120 BaL surface corresponded to approximately 45 RU, consistent with the amount bound per unit of immobilized ligand in the Biorn study. Our calculated KD for the gp120-12p1 interaction was 1.02 µM, marginally lower than the published value of 3.7 µM. The reasons for the slight difference are likely to be related to the different target gp120s used in these studies. The calculated Rmax for 12p1 binding on a 5000 RU gp120BaL surface was approximately 67 RU, and this value was not exceeded when binding up to 250 µM of 12p1 (well over 100 × the published KD value). Our data thus fit to a direct binding model consistent with 1:1 stoichiometry, and closely match previously published results. Collectively, these results demonstrate that Hel-C is able to bind recombinant gp120, and that this binding is likely to involve the formation of a disulphide bond.

### EXAMPLE 4

Hel-C binds at the CD4 binding site on gp120. The EMSA and SPR binding assays demonstrated that Hel-C binds gp120 through disulphide bond formation; indeed, a small amount of the cysteine-containing control peptide Hel-Cs was found to bind gp120. We therefore sought to gain insight into the sites on gp120 to which these peptides were binding, and whether the binding of Hel-C to gp120 could be targeted, as designed, to part of the CD4-gp120 binding interface between the V1/V2 loop stem and CD4-binding β15 strand. Our strategy involved establishing a competition between the peptides and a panel of gp120 monoclonal antibodies, the epitopes of which are well-characterized. We selected two antibodies directed at the CD4 binding site (CD4bs) on gp120 (b12 and 654-30D) and two antibodies that bind at positions independent of CD4bs on gp120 (F425 B4a1 and 2G12). B4a1 binds the V3 loop distal to the CD4-gp120 binding interface, while 2G12 binds glycosylated residues on the same surface to which CCR5/CXCR4 coreceptors attach, and is orthogonal to the CD4bs. We would therefore expect that molecules targeted to the CD4bs might antagonize the binding of the CD4bs-directed MAbs but would leave the binding of CD4bs-independent MAbs largely unhindered. To prove the principle of this approach, we tested the competition between each of these four MAbs and 2-domain CD4 (2dCD4), which contains the two N-terminal domains of CD4 involved in gp120 binding. Consistent with their respective binding sites on gp120, we found that 2dCD4 successfully inhibited, in a dose-dependent manner, the binding of MAbs b12 and 654-30D to gp120. In contrast, the binding of MAbs B4a1 and 2G12 was unaffected at the same concentrations of 2dCD4 tested. We thus proceeded to check whether Hel-C would have a similar effect on the binding of these MAbs to gp120, as might be expected if the CD4-mimetic peptide bound in a manner analogous to the R59-Q64 region of CD4. Interestingly, Hel-C appeared to mediate the same dose-dependent inhibitory effect on the binding of the CD4bs-directed MAbs b12 and 654-30D, while it did not compete with MAbs B4a1 and 2G12. At the same concentrations, the wild-type CD4-mimetic peptide Hel-S and scrambled peptide Hel-Cs did not compete with the binding of any of the 4 MAbs assayed. These data suggest that the binding of Hel-C to gp120 is targeted to the CD4bs on gp120, at a region overlapping the b 12 and 654-30D epitopes, and that the S60C mutation enhances the affinity of Hel-C for gp120. To qualify these findings further, we superimposed the models of Kwong *et al.* (of gp120 in complex with CD4 and the neutralizing antibody 17b) and Zhou *et al.* (of gp120 in complex with b12) to evaluate whether there was a structural basis for the observed competition between b12 and Hel-C. In this model, R58-Q64 of the CD4 D1 chain can be seen occupying a position that coincides with the base of the CDRH2 loop (F59/S60 and G49 to Y53) of b12. Other important coincidental positions between R58-Q64 of CD4 and b12 include the efferent loop of the b12 CDRH1 chain at H35/W36, and T68/F69 on the β-sheet covering the 3 CDR loops. These stereochemical conflicts are proposed to be the basis for the competition observed between Hel-C and b 12. Notably, Hel-S (and the control peptide Hel-Cs) does not compete with b12 for gp120 binding, suggesting that the Ser/Cys substitution is critical for enabling competition between the small 7-mer peptide and b12, which has a demonstrably high affinity for the wild-type gp120 core (approximate KD = 2 × 10-8 M). Collectively these data suggest that Hel-C associates with gp120 at a position where novel disulphide bond formation may be involved in occlusion of the b12-binding site.

To test this hypothesis further, we checked to see whether lowering the oxidation potential of the Hel-C cysteine would influence the ability of the peptide to compete with b12 and 2G12 for gp120 binding. Our approach was to perform the same antibody-Hel-C competition assays under reducing conditions, comparing the extent to which the MAbs competed with Hel-C in the presence and absence of either β-mercaptoethanol or DTT. In order to achieve this, we first needed to check whether the antibodies would indeed bind gp120 at all under these circumstances, so that we could reasonably assess the corresponding effect on their competition with Hel-C. MAbs b12 and 2G12 were added to immobilized gp120BaL in the presence of 0 to 200 mM of either reducing compound, and the percentage of bound antibody quantified spectrophotometrically following chromogenic development of the microtiter plates. Interestingly, b12 and 2G12 are able to bind immobilized gp120 even in the presence of remarkably high concentrations of β-ME and DTT with approximately 50% of the levels of 2G12 and b12 binding seen under native conditions achievable when the reactions were performed in 25 mM and 100 mM of DTT and β-mercaptoethanol, respectively. We thus performed the MAb-Hel-C competition experiments having pre-treated the peptides with 20 mM of β-mercaptoethanol or DTT, and showed that these compounds specifically relieved the Hel-C-mediated inhibition of b12-gp120 binding, while they had no effect on the amount of 2G12 bound in the presence or absence of peptide. Notably, the extent of this relief was complete in the presence of DTT, while β-mercaptoethanol only partially rescued b12 binding. These data are consistent with the respective reduction potentials of the two reducing compounds, and are interpreted as confirmatory evidence that oxidative disulphide bond formation between Hel-C and gp120 at a position overlapping the b12 binding site is involved in occlusion of this antibody epitope.

### EXAMPLE 5

For initial exploration in this area we prepared several short 7 amino acid CD4 mimetic peptides that correspond to the amino acids 83-89 of SEQ ID NO:22 (amino acids 58-63 of the mature CD4 polypeptide). These peptides, when modeled using Swiss PDF viewer with crystal structures of gp120, suggested they may provide a minimal peptide sequence necessary for localization and binding to the CD4 binding site on gp120. These peptides include RRSLWDQ (SEQ ID NO:10, corresponding to the native sequence CD4 which has a serine at position 85 of SEQ ID NO:22), RRCLWDQ (SEQ ID NO:2, where the native serine is substituted with a cysteine, S85C), RRULWDQ (SEQ ID NO:3, where the native serine is substituted with a selenocysteine, S85U) and a peptide for control purposes of scrambled amino acid sequence DLRQCWR (SEQ ID NO:27).

To date, we have shown that all the peptides enhance the binding of gp120 to CD4 in a CD4-gp120 competition ELISA, suggesting that they may be inducing the gp120 transition state complex necessary for CD4 binding. Peptides containing S85C or S85U mutations appear to block the binding of the CD4bs-targeted b12 antibody to gp120 in an epitope occlusion ELISA, whereas the native or scrambled CD4 peptide sequence does not. This suggests that the mutated peptides bind gp120 at the CD4 binding site. Surface plasmon resonance studies investigating binding kinetics between gp120 and peptides suggest that the S85C mutated peptide binds covalently to both monomeric and trimeric gp120 proteins. Additional proof of covalent binding using C14-radioactively labeled S85C mutated peptide in an electrophoretic mobility shift assay has shown it binds covalently to gp120 whereas unmutated peptide does not. In this assay, gp120-bound peptides are resolved on denaturing gels in the absence and presence of different concentrations of reducing agents.

## Claims

1. A polypeptide comprising the amino acid sequence
Arg -Arg-Xaa1-Leu-Xaa2-Asp-Gln
wherein Xaa₁ is Cys or Sec; and Xaa₂ is Trp or Phe; and the polypeptide binds gp120 or competes with an antibody that specifically binds the gp120 binding site of CD4;
with the proviso that the polypeptide is not a wild type CD4 protein or a fragment thereof.

2. The polypeptide of claim 1, wherein the polypeptide comprises the amino acid sequence:
Arg-Arg-Cys-Leu-Trp-Asp-Gln;
Arg-Arg-Sec-Leu-Trp-Asp-Gln;
Arg-Arg-Sec-Leu-Phe-Asp-Gln; or
Arg-Arg-Cys-Leu-Phe-Asp-Gln.

3. The polypeptide of either of claims 1 or 2, wherein the polypeptide further comprises amino acid residues 1-57 of a mature wild type CD4 at the polypeptide's amino terminus and amino acid residues 65-433 of a mature wild type CD4 at the polypeptide's carboxy terminus.

4. The polypeptide of any of claims 1-3, wherein the polypeptide further comprises one or more heterologous amino acid residues at the carboxy- or amino -terminus of the polypeptide; and wherein the one or more heterologous amino acid residues comprise a B cell or T cell epitope, or an immunogenic epitope as a target for an antibody.

5. A complex comprising the polypeptide of any of claims 1-4, in non-linear association with a heterologous polypeptide, wherein the heterologous polypeptide is gp120 or a portion thereof.

6. The complex of claim 5, wherein the polypeptide and the heterologous polypeptide are associated by covalent bonds, wherein the covalent bonds are selected from the group consisting of disulphide, trisulphide, selenide or selenosulphide bonds.

7. The polypeptide of any of claims 1-4, or the complex of either of claims 5 or 6, wherein the side chain of one or more of the amino acid residues within the polypeptide is modified, wherein the modification is glycosylation or pegylation of the one or more amino acid residues.

8. An isolated nucleic acid comprising a nucleotide sequence encoding the polypeptide of any of claims 1-4 or 7.

9. An expression vector comprising the isolated nucleic acid sequence of claim 8.

10. A pharmaceutical composition comprising the polypeptide of any of claims 1-4 or 7, the complex of either of claims 5 or 6, the nucleic acid of claim 8, or the vector of claim 9.

11. A method of preparing blood or a fraction thereof for administration to a subject, the method comprising mixing the blood with an amount of the polypeptide of any of claims 1-4, wherein the amount of the polypeptide is sufficient to reduce the risk of transmission of any HIV that may be present in the blood or the fraction thereof.

12. A kit comprising the polypeptide of any of claims 1-4 and instructional materials for use.

13. A method of detecting gp120, the method comprising:
(a) providing a sample;
(b) exposing the sample to the CD4 polypeptide of any of claims 1-4 for a time and under conditions sufficient to allow binding between any gp120 in the sample and the CD4 polypeptide; and
(c) determining whether the sample includes gp120 by detecting gp120 bound to the CD4 polypeptide.

14. A method of purifying gp120, a gp120-containing complex, or a gp120-expressing virus, the method comprising:
(a) providing a sample;
(b) exposing the sample to the CD4 polypeptide of any of claims 1-4 for a time and under conditions sufficient to allow binding between any gp120 in the sample and the CD4 polypeptide; and
(c) purifying any gp120, gp120-containing complexes, or gp120-expressing viruses in the sample by virtue of removing any gp120 bound to the CD4 polypeptide from the majority of the remaining sample.

## Patentansprüche

1. Polypeptid, umfassend die Aminosäuresequenz
Arg-Arg-Xaal-Leu-Xaa2-Asp-Gln
wobei es sich bei Xaa₁ um Cys oder Sec handelt; und es sich bei Xaa₂ um Trp oder Phe handelt; und das Polypeptid gp120 bindet oder mit einem Antikörper konkurriert, der die gp120-Bindungsstelle von CD4 spezifisch bindet;
unter der Voraussetzung, dass es sich bei dem Polypeptid nicht um ein CD4-Wildtypprotein oder ein Fragment davon handelt.

2. Polypeptid nach Anspruch 1, wobei das Polypeptid die folgende Aminosäuresequenz umfasst:
Arg-Arg-Cys-Leu-Trp-Asp-Gln;
Arg-Arg-Sec-Leu-Trp-Asp-Gln;
Arg-Arg-Sec-Leu-Phe-Asp-Gln; oder
Arg-Arg-Cys-Leu-Phe-Asp-Gln.

3. Polypeptid nach Anspruch 1 oder 2, wobei das Polypeptid ferner die Aminosäurereste 1-57 eines reifen Wildtyp-CD4 am Aminoterminus des Polypeptids und die Aminosäurereste 65-433 eines reifen Wildtyp-CD4 am Carboxyterminus des Polypeptids umfasst.

4. Polypeptid nach einem der Ansprüche 1 - 3, wobei das Polypeptid ferner mindestens einen heterologen Aminosäurerest am Carboxy- oder Aminoterminus des Polypeptids umfasst, und wobei der mindestens eine heterologe Aminosäurerest ein B-Zell- oder ein T-Zell-Epitop oder ein immunogenes Epitop als Ziel für einen Antikörper umfasst.

5. Komplex, umfassend das Polypeptid nach einem der Ansprüche 1 - 4 in nicht-linearer Assoziation mit einem heterologen Polypeptid, wobei es sich bei dem heterologen Polypeptid um gp120 oder einen Abschnitt davon handelt.

6. Komplex nach Anspruch 5, wobei das Polypeptid und das heterologe Polypeptid durch kovalente Bindungen assoziiert sind, wobei die kovalenten Bindungen aus der Gruppe ausgewählt sind, die aus Disulfid, Trisulfid, Selenid oder Selenosulfidbindungen besteht.

7. Polypeptid nach einem der Ansprüche 1 - 4 oder der Komplex nach Anspruch 5 oder 6, wobei die Seitenkette von mindestens einem Aminosäurerest in dem Polypeptid modifiziert ist, wobei es sich bei der Modifikation um Glykosylierung oder Pegylierung des mindestens einen Aminosäurerests handelt.

8. Isolierte Nukleinsäure, umfassend eine Nukleotidsequenz, welche das Polypeptid nach einem der Ansprüche 1 - 4 oder 7 kodiert.

9. Expressionsvektor, umfassend die isolierte Nukleinsäuresequenz nach Anspruch 8.

10. Pharmazeutische Zusammensetzung, umfassend das Polypeptid nach einem der Ansprüche 1 - 4 oder 7, den Komplex nach Anspruch 5 oder 6, die Nukleinsäure nach Anspruch 8 oder den Vektor nach Anspruch 9.

11. Verfahren der Vorbereitung von Blut oder einer Fraktion davon zur Verabreichung an ein Individuum, wobei das Verfahren das Mischen des Bluts mit einer Menge des Polypeptids nach einem der Ansprüche 1 - 4 umfasst, wobei die Menge des Polypeptids ausreicht, um das Risiko der Übertragung jeglicher HIV, die möglicherweise in dem Blut oder der Fraktion davon vorhanden sind, zu reduzieren.

12. Kit, umfassend das Polypeptid nach einem der Ansprüche 1 - 4 und Material mit Anweisungen zum Gebrauch.

13. Verfahren des Detektierens von gp120, wobei das Verfahren Folgendes umfasst:
(a) Bereitstellen einer Probe;
(b) Aussetzen der Probe dem CD4-Polypeptid nach einem der Ansprüche 1 - 4 für einen Zeitraum und unter Bedingungen, der bzw. die ausreichend sind, um eine Bindung zwischen jeglichen gp120 in der Probe und dem CD4-Polypeptid zu ermöglichen; und
(c) Feststellen, ob die Probe gp120 enthält, indem an das CD4-Polypeptid gebundenes gp120 detektiert wird.

14. Verfahren des Reinigens von gp120, eines gp120 enthaltenden Komplexes oder eines gp120 exprimierenden Virus, wobei das Verfahren Folgendes umfasst:
(a) Bereitstellen einer Probe;
(b) Aussetzen der Probe dem CD4-Polypeptid nach einem der Ansprüche 1 - 4 für einen Zeitraum und unter Bedingungen, der bzw. die ausreichend sind, um eine Bindung zwischen jeglichen gp120 in der Probe und dem CD4-Polypeptid zu ermöglichen; und
(c) Reinigen von jeglichem gp120, gp120 enthaltenden Komplexen oder gp120 exprimierenden Viren in der Probe durch Entfernen von jeglichem, an das CD4-Polypeptid gebundenem gp120 aus dem Großteil der verbleibenden Probe.

## Revendications

1. Polypeptide comprenant la séquence d'aminoacides
Arg-Arg-Xaa1-Leu-Xaa2-Asp-Gln
où Xaa₁ est Cys ou Sec ; et Xaa₂ est Trp ou Phe ; et le polypeptide lie gp 120 ou entre en compétition avec un anticorps qui lie spécifiquement le site de liaison de gp 120 de CD4 ;
à condition que le polypeptide ne soit pas une protéine CD4 de type sauvage ou un fragment de celle-ci.

2. Polypeptide selon la revendication 1, où le polypeptide comprend la séquence d'aminoacides :
Arg-Arg-Cys-Leu-Trp-Asp-Gln ;
Arg-Arg-Sec-Leu-Trp-Asp-Gln ;
Arg-Arg-Sec-Leu-Phe-Asp-Gln ; ou
Arg-Arg-Cys-Leu-Phe-Asp-Gln.

3. Polypeptide selon l'une quelconque des revendications 1 et 2, où le polypeptide comprend en outre les résidus d'aminoacides 1-57 d'une CD4 de type sauvage mature à l'extrémité amino du polypeptide et les résidus d'aminoacides 65-433 d'une CD4 de type sauvage mature à l'extrémité carboxy du polypeptide.

4. Polypeptide selon l'une quelconque des revendications 1-3, où le polypeptide comprend en outre un ou plusieurs résidus d'aminoacides hétérologues à l'extrémité carboxy ou amino du polypeptide; et où le un ou plusieurs résidus d'aminoacides hétérologues comprennent un épitope de cellule B ou de cellule T, ou un épitope immunogène comme cible pour un anticorps.

5. Complexe comprenant le polypeptide selon l'une quelconque des revendications 1-4, en association non linéaire avec un polypeptide hétérologue, où le polypeptide hétérologue est gp 120 ou une partie de celle-ci.

6. Complexe selon la revendication 5, où le polypeptide et le polypeptide hétérologue sont associés par des liaisons covalentes, où les liaisons covalentes sont choisies dans le groupe consistant en les liaisons disulfure, trisulfure, séléniure ou sélénosulfure.

7. Polypeptide selon l'une quelconque des revendications 1-4, ou complexe selon l'une quelconque des revendications 5 et 6, où la chaîne latérale d'un ou plusieurs des résidus d'aminoacides dans le polypeptide est modifiée, où la modification est une glycosylation ou une pégylation du un ou plusieurs résidus d'aminoacides.

8. Acide nucléique isolé comprenant une séquence nucléotidique codant le polypeptide selon l'une quelconque des revendications 1-4 ou 7.

9. Vecteur d'expression comprenant la séquence d'acide nucléique isolée selon la revendication 8.

10. Composition pharmaceutique comprenant le polypeptide selon l'une quelconque des revendications 1-4 ou 7, le complexe selon l'une quelconque des revendications 5 et 6, l'acide nucléique selon la revendication 8 ou le vecteur selon la revendication 9.

11. Procédé de préparation de sang ou d'une fraction de celui-ci pour l'administration à un sujet, le procédé comprenant le mélange du sang avec une quantité du polypeptide selon l'une quelconque des revendications 1-4, où la quantité du polypeptide est suffisante pour réduire le risque de transmission de tout VI H qui peut être présent dans le sang ou la fraction de celui-ci.

12. Kit comprenant le polypeptide selon l'une quelconque des revendications 1-4 et des instructions pour l'utilisation.

13. Procédé de détection de gp 120, le procédé comprenant :
(a) la fourniture d'un échantillon ;
(b) l'exposition de l'échantillon au polypeptide CD4 selon l'une quelconque des revendications 1-4 pendant une durée et dans des conditions suffisantes pour permettre une liaison entre toute gp 120 dans l'échantillon et le polypeptide CD4 ; et
(c) la détermination de ce que l'échantillon inclut gp 120 par détection de gp 120 liée au polypeptide CD4.

14. Procédé de purification de gp 120, d'un complexe contenant gp 120 ou d'un virus exprimant gp 120, le procédé comprenant :
(a) la fourniture d'un échantillon ;
(b) l'exposition de l'échantillon au polypeptide CD4 selon l'une quelconque des revendications 1-4 pendant une durée et dans des conditions suffisantes pour permettre une liaison entre toute gp 120 dans l'échantillon et le polypeptide CD4 ; et
(c) la purification de toute gp 120, de tous complexes contenant gp 120 ou de tous virus exprimant gp 120 dans l'échantillon par retrait de toute gp 120 liée au polypeptide CD4 de la majorité de l'échantillon restant.
